# EUROPEAN PATENT APPLICATION

(11) **EP 1 918 299 A1**
(43) Date of publication of application: **07.05.2008**
(21) Application number: 06123480.3
(22) Date of filing: 03.11.2006
(51) Int. Cl.: C07K 14/705, G01N 33/68, A61K 38/00, A61P 25/28, A61P 29/00

(54) **Modulators of cell adhesion molecules and use thereof**

(71) Applicant: UNIVERSITE DE GENEVE, 1211 Genève 4 (CH)
(72) Inventor: Ossipow, Vincent, 1291, Commugny (CH); Pellissier, François, 1227, Carouge (CH); Gerber, Alan, 1205, Geneva (CH); Ballivet, Marc, 1205, Geneva (CH)
(74) Representative: Leman Consulting S.A.

(57) **Abstract**

The present invention is related to new molecules capable of decreasing or altering neuronal cell adhesion and especially cell aggregation induced by Necl/SynCAM molecules. In particular, the invention provides proteins useful in the treatment of neuro-inflammatory and neurodegenerative conditions. More particularly, the present invention provides novel proteins and antibodies, DNA encoding thereof, processes for production thereof, pharmaceutical compositions, kits containing thereof and the use of these in the preparation of pharmaceutical compositions for the treatment of neuroinflammatory and neurodegenerative conditions.

SEQUENCE LISTING

<110>  Université de Genève

<120>  Modulators of cell adhesion molecules and use thereof

<130>  P-54-536-EP

<160>  41

<170>  PatentIn version 3.3

<210>  1
<211>  1308
<212>  DNA
<213>  Homo sapiens

<400>  1
atgatttgga aacgcagcgc cgttctccgc ttctacagtg tctgcgggct cctgctacaa      60

ggcagccaag ggcagtttcc actaacacag aatgtaaccg ttgttgaagg tggaactgca     120

attttgacct gcagggttga tcaaaatgat aacacctccc tccagtggtc aaatccagct     180

caacagactc tgtactttga cgacaagaaa gctttaaggg acaataggat cgagctggtt     240

cgcgcttcct ggcatgaatt gagtattagt gtcagtgatg tgtctctctc tgatgaagga     300

cagtacacct gttctttatt tacaatgcct gtcaaaactt ccaaggcata tctcaccgtt     360

ctgggtgttc ctgaaaagcc tcagattagt ggattctcat caccagttat ggagggtgac     420

ttgatgcagc tgacttgcaa aacatctggt agtaaacctg cagctgatat aagatggttc     480

aaaaatgaca aagagattaa agatgtaaaa tatttaaaag aagaggatgc aaatcgcaag     540

acattcactg tcagcagcac actggacttc cgagtggacc ggagtgatga tggagtggcg     600

gtcatctgca gagtagatca cgaatccctc aatgccaccc ctcaggtagc catgcaggtg     660

ctagaaatac actatacacc atcagttaag attataccat cgactccttt tccacaagaa     720

ggacagcctt taattttgac ttgtgaatcc aaaggaaaac cactgccaga acctgttttg     780

tggacaaagg atggcggaga attaccagat cctgaccgaa tggttgtgag tggtagggag     840

ctaaacattc ttttcctgaa caaaacggat aatggtacat atcgatgtga agccacaaac     900

accattggcc aaagcagtgc ggaatatgtt ctcattgtgc atgatgttcc caacactttg     960

cttcccacta ctatcatccc ctcccttacc actgcaacag tcacaaccac tgtagccata    1020

acaaccagcc caaccacatc tgcaacaacc agcagcatca gagatcctaa tgctttggct    1080

ggccagaatg gccctgacca tgctctcata ggaggaatag tggctgtagt tgtatttgtc    1140

acgctgtgtt ctatctttct gcttggtcga tatctggcaa ggcataaagg aacgtattta    1200

acaaatgaag ctaaaggagc tgaagatgca ccagatgctg atacagccat tatcaatgct    1260

gaaggcagcc aagtcaatgc tgaagagaaa aaagagtatt tcatttaa                 1308


<210>  2
<211>  435
<212>  PRT
<213>  Homo sapiens

<400>  2

Met Ile Trp Lys Arg Ser Ala Val Leu Arg Phe Tyr Ser Val Cys Gly
1               5                   10                  15

Leu Leu Leu Gln Gly Ser Gln Gly Gln Phe Pro Leu Thr Gln Asn Val
            20                  25                  30

Thr Val Val Glu Gly Gly Thr Ala Ile Leu Thr Cys Arg Val Asp Gln
        35                  40                  45

Asn Asp Asn Thr Ser Leu Gln Trp Ser Asn Pro Ala Gln Gln Thr Leu
    50                  55                  60

Tyr Phe Asp Asp Lys Lys Ala Leu Arg Asp Asn Arg Ile Glu Leu Val
65                  70                  75                  80

Arg Ala Ser Trp His Glu Leu Ser Ile Ser Val Ser Asp Val Ser Leu
                85                  90                  95

Ser Asp Glu Gly Gln Tyr Thr Cys Ser Leu Phe Thr Met Pro Val Lys
            100                 105                 110

Thr Ser Lys Ala Tyr Leu Thr Val Leu Gly Val Pro Glu Lys Pro Gln
        115                 120                 125

Ile Ser Gly Phe Ser Ser Pro Val Met Glu Gly Asp Leu Met Gln Leu
    130                 135                 140

Thr Cys Lys Thr Ser Gly Ser Lys Pro Ala Ala Asp Ile Arg Trp Phe
145                 150                 155                 160

Lys Asn Asp Lys Glu Ile Lys Asp Val Lys Tyr Leu Lys Glu Glu Asp
                165                 170                 175

Ala Asn Arg Lys Thr Phe Thr Val Ser Ser Thr Leu Asp Phe Arg Val
            180                 185                 190

Asp Arg Ser Asp Asp Gly Val Ala Val Ile Cys Arg Val Asp His Glu
        195                 200                 205

Ser Leu Asn Ala Thr Pro Gln Val Ala Met Gln Val Leu Glu Ile His
    210                 215                 220

Tyr Thr Pro Ser Val Lys Ile Ile Pro Ser Thr Pro Phe Pro Gln Glu
225                 230                 235                 240

Gly Gln Pro Leu Ile Leu Thr Cys Glu Ser Lys Gly Lys Pro Leu Pro
                245                 250                 255

Glu Pro Val Leu Trp Thr Lys Asp Gly Gly Glu Leu Pro Asp Pro Asp
            260                 265                 270

Arg Met Val Val Ser Gly Arg Glu Leu Asn Ile Leu Phe Leu Asn Lys
        275                 280                 285

Thr Asp Asn Gly Thr Tyr Arg Cys Glu Ala Thr Asn Thr Ile Gly Gln
    290                 295                 300

Ser Ser Ala Glu Tyr Val Leu Ile Val His Asp Val Pro Asn Thr Leu
305                 310                 315                 320

Leu Pro Thr Thr Ile Ile Pro Ser Leu Thr Thr Ala Thr Val Thr Thr
                325                 330                 335

Thr Val Ala Ile Thr Thr Ser Pro Thr Thr Ser Ala Thr Thr Ser Ser
            340                 345                 350

Ile Arg Asp Pro Asn Ala Leu Ala Gly Gln Asn Gly Pro Asp His Ala
        355                 360                 365

Leu Ile Gly Gly Ile Val Ala Val Val Val Phe Val Thr Leu Cys Ser
    370                 375                 380

Ile Phe Leu Leu Gly Arg Tyr Leu Ala Arg His Lys Gly Thr Tyr Leu
385                 390                 395                 400

Thr Asn Glu Ala Lys Gly Ala Glu Asp Ala Pro Asp Ala Asp Thr Ala
                405                 410                 415

Ile Ile Asn Ala Glu Gly Ser Gln Val Asn Ala Glu Glu Lys Lys Glu
            420                 425                 430

Tyr Phe Ile
        435

<210>  3
<211>  1095
<212>  DNA
<213>  Homo sapiens

<400>  3
atgatttgga aacgcagcgc cgttctccgc ttctacagtg tctgcgggct cctgctacaa      60

ggcagccaag ggcagtttcc actaacacag aatgtaaccg ttgttgaagg tggaactgca     120

attttgacct gcagggttga tcaaaatgat aacacctccc tccagtggtc aaatccagct     180

caacagactc tgtactttga cgacaagaaa gctttaaggg acaataggat cgagctggtt     240

cgcgcttcct ggcatgaatt gagtattagt gtcagtgatg tgtctctctc tgatgaagga     300

cagtacacct gttctttatt tacaatgcct gtcaaaactt ccaaggcata tctcaccgtt     360

ctgggtgttc ctgaaaagcc tcagattagt ggattctcat caccagttat ggagggtgac     420

ttgatgcagc tgacttgcaa aacatctggt agtaaacctg cagctgatat aagatggttc     480

aaaaatgaca aagagattaa agatgtaaaa tatttaaaag aagaggatgc aaatcgcaag     540

acattcactg tcagcagcac actggacttc cgagtggacc ggagtgatga tggagtggcg     600

gtcatctgca gagtagatca cgaatccctc aatgccaccc ctcaggtagc catgcaggtg 660

ctagaaatac actatacacc atcagttaag attataccat cgactccttt tccacaagaa 720

ggacagcctt taattttgac ttgtgaatcc aaaggaaaac cactgccaga acctgttttg 780

tggacaaagg atggcggaga attaccagat cctgaccgaa tggttgtgag tggtagggag 840

ctaaacattc ttttcctgaa caaaacggat aatggtacat atcgatgtga agccacaaac 900

accattggcc aaagcagtgc ggaatatgtt ctcattgtgc atgatgttcc caacactttg 960

cttcccacta ctatcatccc ctcccttacc actgcaacag tcacaaccac tgtagccata 1020

acaaccagcc caaccacatc tgcaacaacc agcagcatca gagatcctaa tgctttggct 1080

ggccagaatg gccct 1095

<210> 4
<211> 365
<212> PRT
<213> Homo sapiens

<400> 4

Met Ile Trp Lys Arg Ser Ala Val Leu Arg Phe Tyr Ser Val Cys Gly
1               5                   10                  15

Leu Leu Leu Gln Gly Ser Gln Gly Gln Phe Pro Leu Thr Gln Asn Val
            20                  25                  30

Thr Val Val Glu Gly Gly Thr Ala Ile Leu Thr Cys Arg Val Asp Gln
        35                  40                  45

Asn Asp Asn Thr Ser Leu Gln Trp Ser Asn Pro Ala Gln Gln Thr Leu
    50                  55                  60

Tyr Phe Asp Asp Lys Lys Ala Leu Arg Asp Asn Arg Ile Glu Leu Val
65                  70                  75                  80

Arg Ala Ser Trp His Glu Leu Ser Ile Ser Val Ser Asp Val Ser Leu
                85                  90                  95

Ser Asp Glu Gly Gln Tyr Thr Cys Ser Leu Phe Thr Met Pro Val Lys
            100                 105                 110

Thr Ser Lys Ala Tyr Leu Thr Val Leu Gly Val Pro Glu Lys Pro Gln
        115                 120                 125

Ile Ser Gly Phe Ser Ser Pro Val Met Glu Gly Asp Leu Met Gln Leu
    130                 135                 140

Thr Cys Lys Thr Ser Gly Ser Lys Pro Ala Ala Asp Ile Arg Trp Phe
145                 150                 155                 160

Lys Asn Asp Lys Glu Ile Lys Asp Val Lys Tyr Leu Lys Glu Glu Asp
                165                 170                 175

Ala Asn Arg Lys Thr Phe Thr Val Ser Ser Thr Leu Asp Phe Arg Val
            180                 185                 190

Asp Arg Ser Asp Asp Gly Val Ala Val Ile Cys Arg Val Asp His Glu
        195                 200                 205

Ser Leu Asn Ala Thr Pro Gln Val Ala Met Gln Val Leu Glu Ile His
    210                 215                 220

Tyr Thr Pro Ser Val Lys Ile Ile Pro Ser Thr Pro Phe Pro Gln Glu
225                 230                 235                 240

Gly Gln Pro Leu Ile Leu Thr Cys Glu Ser Lys Gly Lys Pro Leu Pro
                245                 250                 255

Glu Pro Val Leu Trp Thr Lys Asp Gly Gly Glu Leu Pro Asp Pro Asp
            260                 265                 270

Arg Met Val Val Ser Gly Arg Glu Leu Asn Ile Leu Phe Leu Asn Lys
        275                 280                 285

Thr Asp Asn Gly Thr Tyr Arg Cys Glu Ala Thr Asn Thr Ile Gly Gln
    290                 295                 300

Ser Ser Ala Glu Tyr Val Leu Ile Val His Asp Val Pro Asn Thr Leu
305                 310                 315                 320

Leu Pro Thr Thr Ile Ile Pro Ser Leu Thr Thr Ala Thr Val Thr Thr
                325                 330                 335

Thr Val Ala Ile Thr Thr Ser Pro Thr Thr Ser Ala Thr Thr Ser Ser
            340                 345                 350

Ile Arg Asp Pro Asn Ala Leu Ala Gly Gln Asn Gly Pro
        355                 360                 365

<210>  5
<211>  1167
<212>  DNA
<213>  Homo sapiens

<400>  5
atgggccggg cccggcgctt ccagtggccg ctgctgctgc tgtgggcggc cgcggcgggg      60

ccaggggcag gacaggaagt acagacagag aacgtgacag tggctgaggg tggggtggct     120

gagatcacct gccgtctgca ccagtatgat gggtccatag ttgtcatcca gaacccagcc     180

cggcagaccc tcttcttcaa tggcacccgt gccttgaagg atgagcgttt ccagcttgag     240

gagttctccc cacgccgggt gcggatccgg ctctcagatg cccgcctgga ggacgagggg     300

ggctatttct gccagctcta cacagaagac acccaccacc agattgccac gctcacggta     360

ctagtggccc cagagaatcc tgtggtggag gtccgggagc aggcggtaga gggcggcgag     420

gtggagctca gctgcctcgt tccgcggtcc cgtccggctg ccaccctgcg ctggtaccgg 480

gaccgcaagg agctgaaagg agtgagcagc agccaggaaa atggcaaggt ctggagcgtg 540

gcaagcacag tacggtttcg tgtggaccgt aaggacgacg gtggtatcat catctgtgag 600

gcgcagaacc aggcgctgcc ctccggacac agcaagcaga cgcagtacgt gctggatgtg 660

cagtactccc ccacggcccg gattcatgcc tcccaagctg tggtgaggga gggagacacg 720

ctggtgttga cgtgtgctgt cacggggaac cccaggccaa accagatccg ctggaaccgc 780

gggaatgagt ctttgccgga gagggcggag gccgtgggag agacgctcac gctgccgggt 840

ctggtatccg cggataacgg cacctacact tgcgaggcgt ccaataagca cggccatgcg 900

agggcgctct acgtacttgt ggtctacgac cctggtgcgg tggtagaggc tcagacgtcg 960

gttccctatg ccattgtggg cggcatcctg gcgctgctgg tgtttctgat catatgtgtg 1020

ctagtgggca tggtctggtg ctcggtacgg cagaagggtt cctatctgac ccacgaagcc 1080

agtggcttgg atgaacaggg agaagcaaga gaagccttcc tcaatggcag cgacggacac 1140

aagaggaaag aggaattctt catctga 1167

<210> 6
<211> 388
<212> PRT
<213> Homo sapiens

<400> 6

Met Gly Arg Ala Arg Arg Phe Gln Trp Pro Leu Leu Leu Leu Trp Ala
1               5                   10                  15

Ala Ala Ala Gly Pro Gly Ala Gly Gln Glu Val Gln Thr Glu Asn Val
            20                  25                  30

Thr Val Ala Glu Gly Gly Val Ala Glu Ile Thr Cys Arg Leu His Gln
        35                  40                  45

Tyr Asp Gly Ser Ile Val Val Ile Gln Asn Pro Ala Arg Gln Thr Leu
    50                  55                  60

Phe Phe Asn Gly Thr Arg Ala Leu Lys Asp Glu Arg Phe Gln Leu Glu
65                  70                  75                  80

Glu Phe Ser Pro Arg Arg Val Arg Ile Arg Leu Ser Asp Ala Arg Leu
                85                  90                  95

Glu Asp Glu Gly Gly Tyr Phe Cys Gln Leu Tyr Thr Glu Asp Thr His
            100                 105                 110

His Gln Ile Ala Thr Leu Thr Val Leu Val Ala Pro Glu Asn Pro Val
        115                 120                 125

Val Glu Val Arg Glu Gln Ala Val Glu Gly Gly Glu Val Glu Leu Ser
    130                 135                 140

Cys Leu Val Pro Arg Ser Arg Pro Ala Ala Thr Leu Arg Trp Tyr Arg
145                 150                 155                 160

Asp Arg Lys Glu Leu Lys Gly Val Ser Ser Ser Gln Glu Asn Gly Lys
                165                 170                 175

Val Trp Ser Val Ala Ser Thr Val Arg Phe Arg Val Asp Arg Lys Asp
            180                 185                 190

Asp Gly Gly Ile Ile Ile Cys Glu Ala Gln Asn Gln Ala Leu Pro Ser
        195                 200                 205

Gly His Ser Lys Gln Thr Gln Tyr Val Leu Asp Val Gln Tyr Ser Pro
    210                 215                 220

Thr Ala Arg Ile His Ala Ser Gln Ala Val Val Arg Glu Gly Asp Thr
225                 230                 235                 240

Leu Val Leu Thr Cys Ala Val Thr Gly Asn Pro Arg Pro Asn Gln Ile
                245                 250                 255

Arg Trp Asn Arg Gly Asn Glu Ser Leu Pro Glu Arg Ala Glu Ala Val
            260                 265                 270

Gly Glu Thr Leu Thr Leu Pro Gly Leu Val Ser Ala Asp Asn Gly Thr
        275                 280                 285

Tyr Thr Cys Glu Ala Ser Asn Lys His Gly His Ala Arg Ala Leu Tyr
    290                 295                 300

Val Leu Val Val Tyr Asp Pro Gly Ala Val Val Glu Ala Gln Thr Ser
305                 310                 315                 320

Val Pro Tyr Ala Ile Val Gly Gly Ile Leu Ala Leu Leu Val Phe Leu
                325                 330                 335

Ile Ile Cys Val Leu Val Gly Met Val Trp Cys Ser Val Arg Gln Lys
            340                 345                 350

Gly Ser Tyr Leu Thr His Glu Ala Ser Gly Leu Asp Glu Gln Gly Glu
        355                 360                 365

Ala Arg Glu Ala Phe Leu Asn Gly Ser Asp Gly His Lys Arg Lys Glu
    370                 375                 380

Glu Phe Phe Ile
385

<210>  7
<211>  969
<212>  DNA

<213>  Homo sapiens

<400>  7
atgggccggg cccggcgctt ccagtggccg ctgctgctgc tgtgggcggc cgcggcgggg      60

ccaggggcag gacaggaagt acagacagag aacgtgacag tggctgaggg tggggtggct     120

gagatcacct gccgtctgca ccagtatgat gggtccatag ttgtcatcca gaacccagcc     180

cggcagaccc tcttcttcaa tggcacccgt gccttgaagg atgagcgttt ccagcttgag     240

gagttctccc cacgccgggt gcggatccgg ctctcagatg cccgcctgga ggacgagggg     300

ggctatttct gccagctcta cacagaagac acccaccacc agattgccac gctcacggta     360

ctagtggccc cagagaatcc tgtggtggag gtccgggagc aggcggtaga gggcggcgag     420

gtggagctca gctgcctcgt tccgcggtcc cgtccggctg ccaccctgcg ctggtaccgg     480

gaccgcaagg agctgaaagg agtgagcagc agccaggaaa atggcaaggt ctggagcgtg     540

gcaagcacag tacggtttcg tgtggaccgt aaggacgacg gtggtatcat catctgtgag     600

gcgcagaacc aggcgctgcc ctccggacac agcaagcaga cgcagtacgt gctggatgtg     660

cagtactccc ccacggcccg gattcatgcc tcccaagctg tggtgaggga gggagacacg     720

ctggtgttga cgtgtgctgt cacggggaac cccaggccaa accagatccg ctggaaccgc     780

gggaatgagt ctttgccgga gagggcggag gccgtgggag agacgctcac gctgccgggt     840

ctggtatccg cggataacgg cacctacact tgcgaggcgt ccaataagca cggccatgcg     900

agggcgctct acgtacttgt ggtctacgac cctggtgcgg tggtagaggc tcagacgtcg     960

gttccctat                                                             969

<210>  8
<211>  323
<212>  PRT
<213>  Homo sapiens

<400>  8

Met Gly Arg Ala Arg Arg Phe Gln Trp Pro Leu Leu Leu Leu Trp Ala
1               5                   10                  15

Ala Ala Ala Gly Pro Gly Ala Gly Gln Glu Val Gln Thr Glu Asn Val
            20                  25                  30

Thr Val Ala Glu Gly Gly Val Ala Glu Ile Thr Cys Arg Leu His Gln
        35                  40                  45

Tyr Asp Gly Ser Ile Val Val Ile Gln Asn Pro Ala Arg Gln Thr Leu
    50                  55                  60

Phe Phe Asn Gly Thr Arg Ala Leu Lys Asp Glu Arg Phe Gln Leu Glu
65                  70                  75                  80

Glu Phe Ser Pro Arg Arg Val Arg Ile Arg Leu Ser Asp Ala Arg Leu
                85                  90                  95

Glu Asp Glu Gly Gly Tyr Phe Cys Gln Leu Tyr Thr Glu Asp Thr His
            100                 105                 110

His Gln Ile Ala Thr Leu Thr Val Leu Val Ala Pro Glu Asn Pro Val
        115                 120                 125

Val Glu Val Arg Glu Gln Ala Val Glu Gly Gly Glu Val Glu Leu Ser
    130                 135                 140

Cys Leu Val Pro Arg Ser Arg Pro Ala Ala Thr Leu Arg Trp Tyr Arg
145                 150                 155                 160

Asp Arg Lys Glu Leu Lys Gly Val Ser Ser Ser Gln Glu Asn Gly Lys
                165                 170                 175

Val Trp Ser Val Ala Ser Thr Val Arg Phe Arg Val Asp Arg Lys Asp
            180                 185                 190

Asp Gly Gly Ile Ile Ile Cys Glu Ala Gln Asn Gln Ala Leu Pro Ser
        195                 200                 205

Gly His Ser Lys Gln Thr Gln Tyr Val Leu Asp Val Gln Tyr Ser Pro
    210                 215                 220

Thr Ala Arg Ile His Ala Ser Gln Ala Val Val Arg Glu Gly Asp Thr
225                 230                 235                 240

Leu Val Leu Thr Cys Ala Val Thr Gly Asn Pro Arg Pro Asn Gln Ile
                245                 250                 255

Arg Trp Asn Arg Gly Asn Glu Ser Leu Pro Glu Arg Ala Glu Ala Val
            260                 265                 270

Gly Glu Thr Leu Thr Leu Pro Gly Leu Val Ser Ala Asp Asn Gly Thr
        275                 280                 285

Tyr Thr Cys Glu Ala Ser Asn Lys His Gly His Ala Arg Ala Leu Tyr
    290                 295                 300

Val Leu Val Val Tyr Asp Pro Gly Ala Val Val Glu Ala Gln Thr Ser
305                 310                 315                 320

Val Pro Tyr

<210>  9
<211>  3315
<212>  DNA
<213>  Homo sapiens

<400>  9
   gccgctgccg ccacagccgc cgctgcagcc ggagcatccg ggagccgcca ctgccgccgc      60
   cgctgccgct gctaccgcca ctagcgctgc ttccactgct tctacctccc ctcccaggac     120
   cccgagacac cccgggcgcg agcggcagtg ctgcttgctt gctcctcctc tcccccagcc 180
   cttcccctcc gtgacctacc cactccttgc agccctcgcc cgcaccttct ccaacacccc 240
   ggcatccctg caccacctgc tcgggcagcc ccggcgggct ctgggacttg ctgtgcgcgc 300
   cgagaggaag gcaagctcca aacccctgcc tggaagacgg gctgtcgctg ctgcaccacc 360
   agcaggagga ggaggagaag aaactatttc gcgatacccc attctgcggg tgctttgccg 420
   ctgccgcttc tgctgccgcc gatccgagtc cgcgggttcg aacaccgcag cggtggggac 480
   ggtgggtccg gcgggcgccg ggaggaggac accagcggag ccctgcactc tcgtgccccg 540
   ctcaccagca tctacttgcc ccctcgttcc ttccccagcc ctttagagaa gggaccatga 600
   tttggaaacg cagcgccgtt ctccgcttct acagtgtctg cgggctcctg ctacaaggca 660
   gccaagggca gtttccacta acacagaatg taaccgttgt tgaaggtgga actgcaattt 720
   tgacctgcag ggttgatcaa aatgataaca cctccctcca gtggtcaaat ccagctcaac 780
   agactctgta ctttgacgac aagaaagctt taagggacaa taggatcgag ctggttcgcg 840
   cttcctggca tgaattgagt attagtgtca gtgatgtgtc tctctctgat gaaggacagt 900
   acacctgttc tttatttaca atgcctgtca aaacttccaa ggcatatctc accgttctgg 960
   gtgttcctga aaagcctcag attagtggat tctcatcacc agttatggag ggtgacttga 1020
   tgcagctgac ttgcaaaaca tctggtagta aacctgcagc tgatataaga tggttcaaaa 1080
   atgacaaaga gattaaagat gtaaaatatt taaaagaaga ggatgcaaat cgcaagacat 1140
   tcactgtcag cagcacactg gacttccgag tggaccggag tgatgatgga gtggcggtca 1200
   tctgcagagt agatcacgaa tccctcaatg ccacccctca ggtagccatg caggtgctag 1260
   aaatacacta tacaccatca gttaagatta taccatcgac tccttttcca caagaaggac 1320
   agcctttaat tttgacttgt gaatccaaag gaaaaccact gccagaacct gttttgtgga 1380
   caaaggatgg cggagaatta ccagatcctg accgaatggt tgtgagtggt agggagctaa 1440
   acattctttt cctgaacaaa acggataatg gtacatatcg atgtgaagcc acaaacacca 1500
   ttggccaaag cagtgcggaa tatgttctca ttgtgcatga tgttcccaac actttgcttc 1560
   ccactactat catcccctcc cttaccactg caacagtcac aaccactgta gccataacaa 1620
   ccagcccaac cacatctgca acaaccagca gcatcagaga tcctaatgct ttggctggcc 1680
   agaatggccc tgaccatgct ctcataggag gaatagtggc tgtagttgta tttgtcacgc 1740
   tgtgttctat ctttctgctt ggtcgatatc tggcaaggca taaaggaacg tatttaacaa 1800
   atgaagctaa aggagctgaa gatgcaccag atgctgatac agccattatc aatgctgaag 1860
   gcagccaagt caatgctgaa gagaaaaaag agtatttcat ttaagatgca ggccaagatt 1920
   ctgagtttta ctaccaggct gaatgctgga gaaaactggc tatcatcttt cagaagtcat 1980
   ttctaccatc gtctgctacc cttattaact cccatactgt actgctatca gtagccagtg 2040
   tataccaaca atcagctgtt gaaagcatca ttctttaatt actgtaccat ccataatgca 2100
   ggacatttct tactgcctaa atttcacacc attgctcttt taacatacag tgcttgaata 2160
   tacagcctta acaatgttaa tcatctcctt ggatcattat attgagtggt ttttatacat 2220
   taaaaaatgt atgcagagtt tttttccccc attttttccc ctttaagtca tagaccttat 2280
   cagtttgcca ttggacagct ttcacaagta acaggattag ggaaggacat aatgtattta 2340
   agaataagtg aaggttagag gtttacaatt aatgttttct acatgtctgt cttcaaagac 2400
   aacatgcttt ggaaacatat gtcctagaaa acataaaatt aaaaaaaaac actatagtgt 2460
   agtttttgga atgttggaga ttatactggt aaagctgtat cgatttgcta ttgaaatata 2520
   gtatttgata caggagccat gtgtacgaat tgcaatcatg acatggtatt ttctataatt 2580
   atagagatct tactattagg atattgtagc ttcatttacc aataaattat tccaaatgat 2640
   ttactgcttt tggaatcaca aatatttaat tcatttctct aatttgattc tataatttat 2700
   ttgcttcata aagattcacc tgcctaaaca atattgaagt atccataggg cacaattttc 2760
   agacatttta gtatctgtat atagtgcata taataaatcc aattaaacat tatttgatac 2820
   atatttaact tttttggctg tatgtaattc agtcataagt aagcattttc taacgtccat 2880
   tatatccctt taggtattac ttaaaccaat attataagta gataacatgt attagtattt 2940
   ttgtctgtat gtcctagcac tgttcaacaa caaatttttc tagttcttgt taatttttat 3000
   ttgttataca atggaagcac aatgttataa ggaaaggtaa ttttaagcta acaaccagtg 3060
   cacagcctca ggttttaaat tacaaccaca gttgaataat aacctaaaaa taaattttag 3120
   tttgctaaaa atttacaaat tttcattttg cagttccaga gctgcttacc tatgttggtt 3180
   tgccaaaaag aagtgtttaa gatatgtttt ctgtataaat gaactaattc tttatattaa 3240
   aaaaaaaaaa aaaaaaaaaa aaaaaaaaaa aaaaaaaaaa aaaaaaaaaa aaaaaaaaaa 3300
   aaaaaaaaaa aaaaa 3315

<210> 10
<211> 22
<212> DNA
<213> Artificial

<220>
<223> Description of Artificial sequence: primer

<400> 10
ggaattcatg atttggaaac gc 22

<210> 11
<211> 25
<212> DNA
<213> Artificial

<220>
<223> Description of Artificial sequence: primer

<400> 11
ccccaattgc aatgaaatac tcttt 25

<210> 12
<211> 14
<212> DNA
<213> Artificial

<220>
<223>  Description of artificial sequence : primer

<400>  12
aggtctatat aagc                                                          14


<210>  13
<211>  23
<212>  DNA
<213>  Artificial

<220>
<223>  Description of Artificial Sequence: primer

<400>  13
ggggtaccag ggccattctg gcc                                                23


<210>  14
<211>  597
<212>  DNA
<213>  Homo sapiens

<400>  14
aaatatttaa aagaagagga tgcaaatcgc aagacattca ctgtcagcag cacactggac      60

ttccgagtgg accggagtga tgatggagtg gcggtcatct gcagagtaga tcacgaatcc     120

ctcaatgcca cccctcaggt agccatgcag gtgctagaaa tacactatac accatcagtt     180

aagattatac catcgactcc ttttccacaa gaaggacagc ctttaatttt gacttgtgaa     240

tccaaaggaa aaccactgcc agaacctgtt ttgtggacaa aggatggcgg agaattacca     300

gatcctgacc gaatggttgt gagtggtagg gagctaaaca ttcttttcct gaacaaaacg     360

gataatggta catatcgatg tgaagccaca aacaccattg gccaaagcag tgcggaatat     420

gttctcattg tgcatgatgt tcccaacact ttgcttccca ctactatcat cccctccctt     480

accactgcaa cagtcacaac cactgtagcc ataacaacca gcccaaccac atctgcaaca     540

accagcagca tcagagatcc taatgctttg gctggccaga atggccctga ccatgct        597


<210>  15
<211>  198
<212>  PRT
<213>  Homo sapiens

<400>  15

Lys Tyr Leu Lys Glu Glu Asp Ala Asn Arg Lys Thr Phe Thr Val Ser
1               5                   10                  15


Ser Thr Leu Asp Phe Arg Val Asp Arg Ser Asp Asp Gly Val Ala Val
            20                  25                  30


Ile Cys Arg Val Asp His Glu Ser Leu Asn Ala Thr Pro Gln Val Ala
        35                  40                  45


Met Gln Val Leu Glu Ile His Tyr Thr Pro Ser Val Lys Ile Ile Pro
    50                  55                  60

Ser Thr Pro Phe Pro Gln Glu Gly Gln Pro Leu Ile Leu Thr Cys Glu
65                  70                  75                  80

Ser Lys Gly Lys Pro Leu Pro Glu Pro Val Leu Trp Thr Lys Asp Gly
                85                  90                  95

Gly Glu Leu Pro Asp Pro Asp Arg Met Val Val Ser Gly Arg Glu Leu
            100                 105                 110

Asn Ile Leu Phe Leu Asn Lys Thr Asp Asn Gly Thr Tyr Arg Cys Glu
        115                 120                 125

Ala Thr Asn Thr Ile Gly Gln Ser Ser Ala Glu Tyr Val Leu Ile Val
    130                 135                 140

His Asp Val Pro Asn Thr Leu Leu Pro Thr Thr Ile Ile Pro Ser Leu
145                 150                 155                 160

Thr Thr Ala Thr Val Thr Thr Thr Val Ala Ile Thr Thr Ser Pro Thr
                165                 170                 175

Thr Ser Ala Thr Thr Ser Ser Ile Arg Asp Pro Asn Ala Leu Ala Gly
            180                 185                 190

Gln Asn Gly Pro Asp His
        195

<210>  16
<211>  18
<212>  DNA
<213>  Artificial

<220>
<223>  Description of Artificial Sequence: primer

<400>  16
tgaccatgct ctcatagg                                                    18

<210>  17
<211>  18
<212>  DNA
<213>  Artificial

<220>
<223>  Description of artificial sequence: primer

<400>  17
tgccagatat cgaccaag                                                    18

<210>  18
<211>  16
<212>  DNA
<213>  Artificial

<220>
<223>  Description of Artificial Sequence: primer

<400>  18
tgattctacc cacggc                                                       16


<210>  19
<211>  21
<212>  DNA
<213>  Artificial

<220>
<223>  Description of Artificial Sequence: primer

<400>  19
tgatgggttt cccattgatg a                                                 21


<210>  20
<211>  2173
<212>  DNA
<213>  Artificial

<220>
<223>  Description of Artificial Sequence: mutated clone

<400>  20
gcgagcggcg gcggcggcgg cggcggcacc atgggccggg cccggcgctt ccagtggccg      60

ctgctgctgc tgtgggcggc cgcggcgggg ccaggggcag gacaggaagt acagacagag     120

aacgtgacag tggctgaggg tggggtggct gagatcacct gccgtctgca ccagtatgat     180

gggtccatag ttgtcatcca gaacccagcc cggcagaccc tcttcttcaa tggcacccgt     240

gccttgaagg atgagcgttt ccagcttgag gagttctccc cacgccgggt gcggatccgg     300

ctctcagatg cccgcctgga ggacgagggg ggctatttct gccagctcta cacagaagac     360

acccaccacc agattgccac gctcacggta ctagtggccc cagagaatcc tgtggtggag     420

gtccgggagc aggcggtaga gggcggcgag gtggagctca gctgcctcgt tccgcggtcc     480

cgtccggctg ccaccctgcg ctggtaccgg gaccgcaagg agctgaaagg agtgagcagc     540

agccaggaaa atggcaaggt ctagagcgtg gcaagcacag tacggtttcg tgtggaccgt     600

aaggacgacg gtggtatcat catctgtgag gcgcagaacc aggcgctgcc ctccggacac     660

agcaagcaga cgcagtacgt gctggatgtg cagtactccc ccacggcccg gattcatgcc     720

tcccaagctg tggtgaggga gggagacacg ctggtgttga cgtgtgctgt cacggggaac     780

cccaggccaa accagatccg ctggaaccgc gggaatgagt ctttgccgga gagggcggag     840

gccgtgggag agacgctcac gctgccgggt ctggtatccg cggataacgg cacctacact     900

tgcgaggcgt ccaataagca cggccatgcg agggcgctct acgtacttgt ggtctacgac     960

cctggtgcgg tggtagaggc tcagacgtcg gttccctatg ccattgtggg cggcatcctg    1020

gcgctgctgg tgtttctgat catatgtgtg ctagtgggca tggtctggtg ctcggtacgg    1080

cagaagggtt cctatctgac ccacgaagcc agtggcttgg atgaacaggg agaagcaaga    1140

gaagccttcc tcaatggcag cgacggacac aagaggaaag aggaattctt catctgaccc    1200

tatccccacc ccaggcctag gcctgggcct gggctggggt cccccccact gccagctgca    1260

aggaaccagc aaagacattt accagagtct gggatggtgg gcttctcccc ccaccactaa    1320

cacctcagac gcttgggcag ggatgggggt gttggatgcc tggatctctg taagggccag 1380

aagtgagggc ccagaggtct gggtccccca gggggcaggg gccaaaggtc cagacccccc 1440

aagtccagtg agggcagtag ggattgggtt gggggaagat aactggggga aggccagggc 1500

cactaggcta caaaaccagg tcttgtgggg agggggtcag tttctgggaa gggtgggggg 1560

ggcagggaag gggaacacag atttctttgg gggtcctaga ccccatgcca gccattgtaa 1620

gagttccaca gagctctggg cacttctttg caaagccatg tttgcacggt ggggggatgg 1680

gtggggggag ggcgtggaaa tagggattct gtgtctttgt gtcataactt tgatgggggc 1740

agtgagggag acagccccaa cccttttctc caatccccct tccccaggtt cctgggctcc 1800

ccttctctct accttctccc ccaacgtctg tcccatccat atttgtctct ctgtccaccc 1860

actcctgggg gggccttccc catctctcct ccaggccccc cggggagggg gaaaggagtt 1920

tggggaggat tcgtggtctt catggtttta tatataatat attaaaaaat caaaagtctg 1980

tatgaaaata tcagattgca cccccctctc cccattcctg gcttttgccc cctttttctt 2040

gttaaaaaac aaaaaacaaa aaacaaaaaa ccacacacac attttgtacg gggtggggag 2100

gggaatgggg agggggtttg gcaatctcac taaccaccat taaactgagg agagaacaaa 2160

aaaaaaaaaa aaa 2173

<210> 21
<211> 24
<212> DNA
<213> Artificial

<220>
<223> Description of Artificial Sequence: Primer

<400> 21
ccccaattga tgggccgggc ccgg 24

<210> 22
<211> 38
<212> DNA
<213> Artificial

<220>
<223> Description of Artificial Sequence: Primer

<400> 22
ccccagatct aagatgaaga attcctcttt cctcttgt 38

<210> 23
<211> 27
<212> DNA
<213> Artificial

<220>
<223> Definition of Artificial sequence: primer

<400> 23
cccgcatgca atcctgtggt ggaggtc 27

<210> 24
<211> 25

<212>  DNA
<213>  Artificial

<220>
<223>  Definition of artificial sequence: primer

<400>  24
cccctgcagg ggaaccgacg tctga                                          25


<210>  25
<211>  591
<212>  DNA
<213>  Homo sapiens

<400>  25
aatcctgtgg tggaggtccg ggagcaggcg gtagagggcg gcgaggtgga gctcagctgc      60

ctcgttccgc ggtcccgtcc ggctgccacc ctgcgctggt accgggaccg caaggagctg     120

aaaggagtga gcagcagcca ggaaaatggc aaggtctgga gcgtggcaag cacagtacgg     180

tttcgtgtgg accgtaagga cgacggtggt atcatcatct gtgaggcgca gaaccaggcg     240

ctgccctccg gacacagcaa gcagacgcag tacgtgctgg atgtgcagta ctcccccacg     300

gcccggattc atgcctccca agctgtggtg agggagggag acacgctggt gttgacgtgt     360

gctgtcacgg ggaaccccag gccaaaccag atccgctgga accgcgggaa tgagtctttg     420

ccggagaggg cggaggccgt gggagagacg ctcacgctgc cgggtctggt atccgcggat     480

aacggcacct acacttgcga ggcgtccaat aagcacggcc atgcgagggc gctctacgta     540

cttgtggtct acgaccctgg tgcggtggta gaggctcaga cgtcggttcc c              591


<210>  26
<211>  197
<212>  PRT
<213>  Homo sapiens

<400>  26

Asn Pro Val Val Glu Val Arg Glu Gln Ala Val Glu Gly Gly Glu Val
1               5                   10                  15


Glu Leu Ser Cys Leu Val Pro Arg Ser Arg Pro Ala Ala Thr Leu Arg
            20                  25                  30


Trp Tyr Arg Asp Arg Lys Glu Leu Lys Gly Val Ser Ser Ser Gln Glu
        35                  40                  45


Asn Gly Lys Val Trp Ser Val Ala Ser Thr Val Arg Phe Arg Val Asp
    50                  55                  60


Arg Lys Asp Asp Gly Gly Ile Ile Ile Cys Glu Ala Gln Asn Gln Ala
65                  70                  75                  80


Leu Pro Ser Gly His Ser Lys Gln Thr Gln Tyr Val Leu Asp Val Gln
                85                  90                  95


Tyr Ser Pro Thr Ala Arg Ile His Ala Ser Gln Ala Val Val Arg Glu

100                 105                 110

Gly Asp Thr Leu Val Leu Thr Cys Ala Val Thr Gly Asn Pro Arg Pro
        115                 120                 125

Asn Gln Ile Arg Trp Asn Arg Gly Asn Glu Ser Leu Pro Glu Arg Ala
    130                 135                 140

Glu Ala Val Gly Glu Thr Leu Thr Leu Pro Gly Leu Val Ser Ala Asp
145                 150                 155                 160

Asn Gly Thr Tyr Thr Cys Glu Ala Ser Asn Lys His Gly His Ala Arg
                165                 170                 175

Ala Leu Tyr Val Leu Val Val Tyr Asp Pro Gly Ala Val Val Glu Ala
            180                 185                 190

Gln Thr Ser Val Pro
        195

<210>  27
<211>  20
<212>  DNA
<213>  Artificial

<220>
<223>  Description Artificial Sequence: primer

<400>  27
gagggcgctc tacgtacttg                                                   20

<210>  28
<211>  20
<212>  DNA
<213>  Artificial

<220>
<223>  Definition of Artificial Sequence: primer

<400>  28
aaccgacgtc tgagcctcta                                                   20

<210>  29
<211>  1680
<212>  DNA
<213>  Artificial

<220>
<223>  Description Artificial Sequence: clone

<400>  29
gtcaacatcg tagtccaccc cctccccatc cccagccccc ggggattcag gctcgccagc      60

gcccagccag ggagccggcc gggaagcgcg atgggggccc cagccgcctc gctcctgctc     120

ctgctcctgc tgttcgcctg ctgctgggcg cccggcgggg ccaacctctc ccaggacgac     180

agccagccct ggacatctga tgaaacagtg gtggctggtg gcaccgtggt gctcaagtgc     240

caagtgaaag atcacgagga ctcatccctg caatggtcta accctgctca gcagactctc     300

tactttgggg agaagagagc ccttcgagat aatcgaattc agctggttac ctctacgccc 360

cacgagctca gcatcagcat cagcaatgtg gccctggcag acgagggcga gtacacctgc 420

tcaatcttca ctatgcctgt gcgaactgcc aagtccctcg tcactgtgct aggaattcca 480

cagaagccca tcatcactgg ttataaatct tcattacggg aaaaagacac agccacccta 540

aactgtcagt cttctgggag caagcctgca gcccggctca cctggagaaa gggtgaccaa 600

gaactccacg gagaaccaac ccgcatacag gaagatccca atggtaaaac cttcactgtc 660

agcagctcgg tgacattcca ggttacccgg gaggatgatg gggcgagcat cgtgtgctct 720

gtgaaccatg aatctctaaa gggagctgac agatccacct ctcaacgcat tgaagtttta 780

tacacaccaa ctgcgatgat taggccagac cctccccatc ctcgtgaggg ccagaagctg 840

ttgctacact gtgagggtcg cggcaatcca gtcccccagc agtacctatg ggagaaggag 900

ggcagtgtgc cacccctgaa gatgacccag gagagtgccc tgatcttccc tttcctcaac 960

aagagtgaca gtggcaccta cggctgcaca gccaccagca acatgggcag ctacaaggcc 1020

tactacaccc tcaatgttaa tgaccccagt ccggtgccct cctcctccag cacctaccac 1080

gccatcatcg gtgggatcgt ggctttcatt gtcttcctgc tgctcatcat gctcatcttc 1140

ctcggccact acttgatccg gcacaaagga acctacctga cacatgaggc aaaaggctcc 1200

gacgatgctc cagacgcgga cacggccatc atcaatgcag aaggcgggca gtcaggaggg 1260

gacgacaaga aggaatattt catctagagg cgcctgccca cttcctgcgc cccccagggg 1320

ccctgtgggg actgctgggg ccgtcaccaa cccggacttg tacagagcaa ccgcagggcc 1380

gcccctcccg cttgctcccc agcccaccca cccccctgta cagaatgtct gctttgggtg 1440

cggttttgta ctcggtttgg aatggggagg gaggagggcg gggggagggg agggttgccc 1500

tcagcccttt ccgtggcttc tctgcatttg ggttattatt atttttgtaa caatcccaaa 1560

gcaaatctgt ctccaggctg gagaggcagg agccctgggg tgagaaaagc aaaaaacaaa 1620

caaaaaacaa aaaaaaaaaa aaaaaaaaaa aaaaaaaaaa aaaaaaaaaa aaaaaaaaaa 1680

<210> 30
<211> 26
<212> DNA
<213> Artificial

<220>
<223> Description Artificial Sequence: primer

<400> 30
ccccaattga tgggggcccc agccgc 26

<210> 31
<211> 39
<212> DNA
<213> Artificial

<220>
<223> Description Artificial sequence: primer

<400> 31

ccccagatct aagatgaaat attccttctt gtcgtcccc                                39

<210>  32
<211>  1448
<212>  DNA
<213>  Artificial

<220>
<223>  Description artificial sequence: clone

<400>  32
agtctgaggc aggtgcccga catggcgagt gtagtgctgc cgagcggatc ccagtgtgcg      60

gcggcagcgg cggcggcggc gcctcccggg ctccggctcc ggcttctgct gttgctcttc     120

tccgccgcgg cactgatccc cacaggtgat gggcagaatc tgtttacgaa agacgtgaca     180

gtgatcgagg gagaggttgc gaccatcagt tgccaagtca ataagagtga cgactctgtg     240

attcagctac tgaatcccaa caggcagacc atttatttca gggacttcag gcctttgaag     300

gacagcaggt ttcagttgct gaatttttct agcagtgaac tcaaagtatc attgacaaac     360

gtctcaattt ctgatgaagg aagatacttt tgccagctct ataccgatcc cccacaggaa     420

agttacacca ccatcacagt cctggtccca ccacgtaatc tgatgatcga tatccagaaa     480

gacactgcgg tggaaggtga ggagattgaa gtcaactgca ctgctatggc cagcaagcca     540

gccacgacta tcaggtggtt caaagggaac acagagctaa aaggcaaatc ggaggtggaa     600

gagtggtcag acatgtacac tgtgaccagt cagctgatgc tgaaggtgca caaggaggac     660

gatggggtcc cagtgatctg ccaggtggag caccctgcgg tcactggaaa cctgcagacc     720

cagcggtatc tagaagtaca gtataagcct caagtgcaca ttcagatgac ttatcctcta     780

caaggcttaa cccgggaagg ggacgcgctt gagttaacat gtgaagccat cgggaagccc     840

cagcctgtga tggtaacttg ggtgagagtc gatgatgaaa tgcctcaaca cgccgtactg     900

tctgggccca acctgttcat caataaccta aacaaaacag ataatggtac ataccgctgt     960

gaagcttcaa acatagtggg gaaagctcac tcggattata tgctgtatgt atacgatccc    1020

cccacaacta tccctcctcc cacaacaacc accaccacca ccaccaccac caccaccacc    1080

atccttacca tcatcacaga ttcccgagca ggtgaagaag gctcgatcag ggcagtggat    1140

catgccgtga tcggtggcgt cgtggcggtg gtggtgttcg ccatgctgtg cttgctcatc    1200

attctggggc gctattttgc cagacataaa ggtacatact tcactcatga agccaaagga    1260

gccgatgacg cagcagacgc agacacagct ataatcaatg cagaaggagg acagaacaac    1320

tccgaagaaa agaaagagta cttcatctag atcagccttt ttgtttcaat gaggtgtcca    1380

actggcccta tttagatgat aaagagacag tgatattgaa aaaaaaaaaa aaaaaaaaaa    1440

aaaaaaaa                                                             1448

<210>  33
<211>  22
<212>  DNA
<213>  Artificial

<220>

<223>  Description artificial sequence: primer

<400>  33
ggaattcatg gcgagtgtag tg                                                22


<210>  34
<211>  25
<212>  DNA
<213>  Artificial

<220>
<223>  Description artificial sequence: primer

<400>  34
ccccaattgc gatgaagtac tcttt                                             25


<210>  35
<211>  227
<212>  PRT
<213>  Homo sapiens

<400>  35

Asp Lys Thr His Thr Cys Pro Pro Cys Pro Ala Pro Glu Leu Leu Gly
1               5                   10                  15


Gly Pro Ser Val Phe Leu Phe Pro Pro Lys Pro Lys Asp Thr Leu Met
            20                  25                  30


Ile Ser Arg Thr Pro Glu Val Thr Cys Val Val Val Asp Val Ser His
        35                  40                  45


Glu Asp Pro Glu Val Lys Phe Asn Trp Tyr Val Asp Gly Val Glu Val
    50                  55                  60


His Asn Ala Lys Thr Lys Pro Arg Glu Glu Gln Tyr Asn Ser Thr Tyr
65                  70                  75                  80


Arg Val Val Ser Val Leu Thr Val Leu His Gln Asp Trp Leu Asn Gly
                85                  90                  95


Lys Glu Tyr Lys Cys Lys Val Ser Asn Lys Ala Leu Pro Ala Pro Ile
            100                 105                 110


Glu Lys Thr Ile Ser Lys Ala Lys Gly Gln Pro Arg Glu Pro Gln Val
        115                 120                 125


Tyr Thr Leu Pro Pro Ser Arg Glu Glu Met Thr Lys Asn Gln Val Ser
    130                 135                 140


Leu Thr Cys Leu Val Lys Gly Phe Tyr Pro Ser Asp Ile Ala Val Glu
145                 150                 155                 160


Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Gly Leu His Asn His Tyr Thr Gln Lys Ser Leu Ser Leu Ser
    210                 215                 220

Pro Gly Lys
225

<210>  36
<211>  224
<212>  PRT
<213>  Homo sapiens

<400>  36

Pro Pro Cys Pro Ser Cys Pro Ala Pro Glu Phe Leu Gly Gly Pro Ser
1               5                   10                  15

Val Phe Leu Phe Pro Pro Lys Pro Lys Asp Thr Leu Met Ile Ser Arg
            20                  25                  30

Thr Pro Glu Val Thr Cys Val Val Val Asp Val Ser Gln Glu Asp Pro
        35                  40                  45

Glu Val Gln Phe Asn Trp Tyr Val Asp Gly Val Glu Val His Asn Ala
    50                  55                  60

Lys Thr Lys Pro Arg Glu Glu Gln Phe Asn Ser Thr Tyr Arg Val Val
65                  70                  75                  80

Ser Val Leu Thr Val Leu His Gln Asp Trp Leu Asn Gly Lys Glu Tyr
                85                  90                  95

Lys Cys Lys Val Ser Asn Lys Gly Leu Pro Ser Ser Ile Glu Lys Thr
            100                 105                 110

Ile Ser Lys Ala Lys Gly Gln Pro Arg Glu Pro Gln Val Tyr Thr Leu
        115                 120                 125

Pro Pro Ser Gln Glu Glu Met Thr Lys Asn Gln Val Ser Leu Thr Cys
    130                 135                 140

Leu Val Lys Gly Phe Tyr Pro Ser Asp Ile Ala Val Glu Trp Glu Ser
145                 150                 155                 160

Asn Gly Gln Pro Glu Asn Asn Tyr Lys Thr Thr Pro Pro Val Leu Asp
                165                 170                 175

Ser Asp Gly Ser Phe Phe Leu Tyr Ser Arg Leu Thr Val Asp Lys Ser
            180                 185                 190

Arg Trp Gln Glu Gly Asn Val Phe Ser Cys Ser Val Met His Glu Ala
        195                 200                 205

Leu His Asn His Tyr Thr Gln Lys Ser Leu Ser Leu Ser Pro Gly Lys
    210                 215                 220

<210>  37
<211>  227
<212>  PRT
<213>  Mus musculus

<400>  37

Pro Pro Leu Lys Glu Cys Pro Pro Cys Ala Ala Pro Asp Leu Leu Gly
1               5                   10                  15

Gly Pro Ser Val Phe Ile Phe Pro Pro Lys Ile Lys Asp Val Leu Met
            20                  25                  30

Ile Ser Leu Ser Pro Met Val Thr Cys Val Val Val Asp Val Ser Glu
        35                  40                  45

Asp Asp Pro Asp Val Gln Ile Ser Trp Phe Val Asn Asn Val Glu Val
    50                  55                  60

His Thr Ala Gln Thr Gln Thr His Arg Glu Asp Tyr Asn Ser Thr Leu
65                  70                  75                  80

Arg Val Val Ser Ala Leu Pro Ile Gln His Gln Asp Trp Met Ser Gly
                85                  90                  95

Lys Glu Phe Lys Cys Lys Val Asn Asn Arg Ala Leu Pro Ser Pro Ile
            100                 105                 110

Glu Lys Thr Ile Ser Lys Pro Arg Gly Pro Val Arg Ala Pro Gln Val
        115                 120                 125

Tyr Val Leu Pro Pro Pro Ala Glu Glu Met Thr Lys Lys Glu Phe Ser
    130                 135                 140

Leu Thr Cys Met Ile Thr Gly Phe Leu Pro Ala Glu Ile Ala Val Asp
145                 150                 155                 160

Trp Thr Ser Asn Gly Arg Thr Glu Gln Asn Tyr Lys Asn Thr Ala Thr
                165                 170                 175

Val Leu Asp Ser Asp Gly Ser Tyr Phe Met Tyr Ser Lys Leu Arg Val
            180                 185                 190

Gln Lys Ser Thr Trp Glu Arg Gly Ser Leu Phe Ala Cys Ser Val Val
        195                 200                 205

His Glu Gly Leu His Asn His Leu Thr Thr Lys Thr Ile Ser Arg Ser
    210                 215                 220

Leu Gly Lys
225

<210>  38
<211>  232
<212>  PRT
<213>  Mus musculus

<400>  38

Pro Arg Ile Pro Lys Pro Ser Thr Pro Pro Gly Ser Ser Cys Pro Pro
1               5                   10                  15

Gly Asn Ile Leu Gly Gly Pro Ser Val Phe Ile Phe Pro Pro Lys Pro
            20                  25                  30

Lys Asp Ala Leu Met Ile Ser Leu Thr Pro Lys Val Thr Cys Val Val
        35                  40                  45

Val Asp Val Ser Glu Asp Asp Pro Asp Val His Val Ser Trp Phe Val
    50                  55                  60

Asp Asn Lys Glu Val His Thr Ala Trp Thr Gln Pro Arg Glu Ala Gln
65                  70                  75                  80

Tyr Asn Ser Thr Phe Arg Val Val Ser Ala Leu Pro Ile Gln His Gln
                85                  90                  95

Asp Trp Met Arg Gly Lys Glu Phe Lys Cys Lys Val Asn Asn Lys Ala
            100                 105                 110

Leu Pro Ala Pro Ile Glu Arg Thr Ile Ser Lys Pro Lys Gly Arg Ala
        115                 120                 125

Gln Thr Pro Gln Val Tyr Thr Ile Pro Pro Pro Arg Glu Gln Met Ser
    130                 135                 140

Lys Lys Lys Val Ser Leu Thr Cys Leu Val Thr Asn Phe Phe Ser Glu
145                 150                 155                 160

Ala Ile Ser Val Glu Trp Glu Arg Asn Gly Glu Leu Glu Gln Asp Tyr
                165                 170                 175

Lys Asn Thr Pro Pro Ile Leu Asp Ser Asp Gly Thr Tyr Phe Leu Tyr
            180                 185                 190

Ser Lys Leu Thr Val Asp Thr Asp Ser Trp Leu Gln Gly Glu Ile Phe
        195                 200                 205

Thr Cys Ser Val Val His Glu Ala Leu His Asn His His Thr Gln Lys
    210                 215                 220

Asn Leu Ser Arg Ser Pro Gly Lys
225                 230


<210>  39
<211>  398
<212>  PRT
<213>  Homo sapiens

<400>  39

Met Gly Ala Pro Ala Ala Ser Leu Leu Leu Leu Leu Leu Leu Phe Ala
1               5                   10                  15

Cys Cys Trp Ala Pro Gly Gly Ala Asn Leu Ser Gln Asp Asp Ser Gln
            20                  25                  30

Pro Trp Thr Ser Asp Glu Thr Val Val Ala Gly Gly Thr Val Val Leu
        35                  40                  45

Lys Cys Gln Val Lys Asp His Glu Asp Ser Ser Leu Gln Trp Ser Asn
    50                  55                  60

Pro Ala Gln Gln Thr Leu Tyr Phe Gly Glu Lys Arg Ala Leu Arg Asp
65                  70                  75                  80

Asn Arg Ile Gln Leu Val Thr Ser Thr Pro His Glu Leu Ser Ile Ser
                85                  90                  95

Ile Ser Asn Val Ala Leu Ala Asp Glu Gly Glu Tyr Thr Cys Ser Ile
            100                 105                 110

Phe Thr Met Pro Val Arg Thr Ala Lys Ser Leu Val Thr Val Leu Gly
        115                 120                 125

Ile Pro Gln Lys Pro Ile Ile Thr Gly Tyr Lys Ser Ser Leu Arg Glu
    130                 135                 140

Lys Asp Thr Ala Thr Leu Asn Cys Gln Ser Ser Gly Ser Lys Pro Ala
145                 150                 155                 160

Ala Arg Leu Thr Trp Arg Lys Gly Asp Gln Glu Leu His Gly Glu Pro
                165                 170                 175

Thr Arg Ile Gln Glu Asp Pro Asn Gly Lys Thr Phe Thr Val Ser Ser
            180                 185                 190

Ser Val Thr Phe Gln Val Thr Arg Glu Asp Asp Gly Ala Ser Ile Val
        195                 200                 205

Cys Ser Val Asn His Glu Ser Leu Lys Gly Ala Asp Arg Ser Thr Ser
    210                 215                 220

Gln Arg Ile Glu Val Leu Tyr Thr Pro Thr Ala Met Ile Arg Pro Asp
225                 230                 235                 240

Pro Pro His Pro Arg Glu Gly Gln Lys Leu Leu Leu His Cys Glu Gly
                245                 250                 255

Arg Gly Asn Pro Val Pro Gln Gln Tyr Leu Trp Glu Lys Glu Gly Ser
            260                 265                 270

Val Pro Pro Leu Lys Met Thr Gln Glu Ser Ala Leu Ile Phe Pro Phe
        275                 280                 285

Leu Asn Lys Ser Asp Ser Gly Thr Tyr Gly Cys Thr Ala Thr Ser Asn
    290                 295                 300

Met Gly Ser Tyr Lys Ala Tyr Tyr Thr Leu Asn Val Asn Asp Pro Ser
305                 310                 315                 320

Pro Val Pro Ser Ser Ser Ser Thr Tyr His Ala Ile Ile Gly Gly Ile
                325                 330                 335

Val Ala Phe Ile Val Phe Leu Leu Leu Ile Met Leu Ile Phe Leu Gly
            340                 345                 350

His Tyr Leu Ile Arg His Lys Gly Thr Tyr Leu Thr His Glu Ala Lys
        355                 360                 365

Gly Ser Asp Asp Ala Pro Asp Ala Asp Thr Ala Ile Ile Asn Ala Glu
    370                 375                 380

Gly Gly Gln Ser Gly Gly Asp Asp Lys Lys Glu Tyr Phe Ile
385                 390                 395

<210>  40
<211>  442
<212>  PRT
<213>  Homo sapiens

<400>  40

Met Ala Ser Val Val Leu Pro Ser Gly Ser Gln Cys Ala Ala Ala Ala
1               5                   10                  15

Ala Ala Ala Ala Pro Pro Gly Leu Arg Leu Arg Leu Leu Leu Leu Leu
            20                  25                  30

Phe Ser Ala Ala Ala Leu Ile Pro Thr Gly Asp Gly Gln Asn Leu Phe
        35                  40                  45

Thr Lys Asp Val Thr Val Ile Glu Gly Glu Val Ala Thr Ile Ser Cys
    50                  55                  60

Gln Val Asn Lys Ser Asp Asp Ser Val Ile Gln Leu Leu Asn Pro Asn
65                  70                  75                  80

Arg Gln Thr Ile Tyr Phe Arg Asp Phe Arg Pro Leu Lys Asp Ser Arg
                85                  90                  95

Phe Gln Leu Leu Asn Phe Ser Ser Ser Glu Leu Lys Val Ser Leu Thr
            100                 105                 110

Asn Val Ser Ile Ser Asp Glu Gly Arg Tyr Phe Cys Gln Leu Tyr Thr
        115                 120                 125

Asp Pro Pro Gln Glu Ser Tyr Thr Thr Ile Thr Val Leu Val Pro Pro
    130                 135                 140

Arg Asn Leu Met Ile Asp Ile Gln Lys Asp Thr Ala Val Glu Gly Glu
145                 150                 155                 160

Glu Ile Glu Val Asn Cys Thr Ala Met Ala Ser Lys Pro Ala Thr Thr
                165                 170                 175

Ile Arg Trp Phe Lys Gly Asn Thr Glu Leu Lys Gly Lys Ser Glu Val
            180                 185                 190

Glu Glu Trp Ser Asp Met Tyr Thr Val Thr Ser Gln Leu Met Leu Lys
        195                 200                 205

Val His Lys Glu Asp Asp Gly Val Pro Val Ile Cys Gln Val Glu His
    210                 215                 220

Pro Ala Val Thr Gly Asn Leu Gln Thr Gln Arg Tyr Leu Glu Val Gln
225                 230                 235                 240

Tyr Lys Pro Gln Val His Ile Gln Met Thr Tyr Pro Leu Gln Gly Leu
                245                 250                 255

Thr Arg Glu Gly Asp Ala Leu Glu Leu Thr Cys Glu Ala Ile Gly Lys
            260                 265                 270

Pro Gln Pro Val Met Val Thr Trp Val Arg Val Asp Asp Glu Met Pro
        275                 280                 285

Gln His Ala Val Leu Ser Gly Pro Asn Leu Phe Ile Asn Asn Leu Asn
    290                 295                 300

Lys Thr Asp Asn Gly Thr Tyr Arg Cys Glu Ala Ser Asn Ile Val Gly
305                 310                 315                 320

Lys Ala His Ser Asp Tyr Met Leu Tyr Val Tyr Asp Pro Pro Thr Thr
                325                 330                 335

Ile Pro Pro Pro Thr Thr Thr Thr Thr Thr Thr Thr Thr Thr Thr Thr
            340                 345                 350

Thr Ile Leu Thr Ile Ile Thr Asp Ser Arg Ala Gly Glu Glu Gly Ser
        355                 360                 365

Ile Arg Ala Val Asp His Ala Val Ile Gly Gly Val Val Ala Val Val
    370                 375                 380

Val Phe Ala Met Leu Cys Leu Leu Ile Ile Leu Gly Arg Tyr Phe Ala
385                 390                 395                 400

Arg His Lys Gly Thr Tyr Phe Thr His Glu Ala Lys Gly Ala Asp Asp
                405                 410                 415

Ala Ala Asp Ala Asp Thr Ala Ile Ile Asn Ala Glu Gly Gly Gln Asn
            420                 425                 430

Asn Ser Glu Glu Lys Lys Glu Tyr Phe Ile
        435                 440

<210>  41
<211>  21
<212>  PRT
<213>  Artificial

<220>
<223>  Synthetic peptide

<400>  41

Met Glu Val Gly Trp Tyr Arg Ser Pro Phe Ser Arg Val Val His Leu
1               5                   10                  15

Tyr Arg Asn Gly Lys
            20

## Description

### Field of the Invention

The present invention relates to new molecules capable of decreasing or altering cell adhesion and especially cell aggregation induced by Necl (Nectin-like)/SynCAM (Synaptic cell adhesion) molecules and their ligands such as other members of the Necl protein family. In particular, the invention provides proteins useful in the treatment of CNS (central nervous system) disorders, including neuro-inflammatory and neurodegenerative conditions. More particularly, the present invention provides novel proteins and antibodies, DNA encoding thereof, processes for production thereof, pharmaceutical compositions, kits containing thereof and use of these in the preparation of pharmaceutical compositions for the treatment of neuroinflammatory and neurodegenerative conditions.

### Background of the Invention

Neurodegenerative conditions are characterized by a deterioration or loss of function of nerve cells and other cells of the central nervous system (CNS), which may include injuries or damages at the level of the axons and of synaptic connections. The deterioration of nerve cell function through the progressive destruction of the myelin sheath that protects the nerves and allows uninterrupted transmission of nerve impulses is also called demyelination.

Multiple sclerosis (MS) is the most known chronic inflammatory demyelinating disease of the central nervous system in humans. The onset of the disease typically occurs during ages 20 to 40. Women are affected approximately twice as often as men. It is estimated to affect about 2.5 million people worldwide and its economic impact is considerable.

This chronic disease is typically characterised by episodes of neurological deficit followed by periods of remission. MS is manifested in physical symptoms (relapses and disability progression), Central Nervous System (CNS) inflammation, brain atrophy and cognitive impairment. Presenting symptoms include focal sensory deficits, focal weakness, visual problems, imbalance and fatigue. Sexual impairment and sphincter dysfunction may occur.

Approximately half of the patients with MS may experience cognitive impairment or depression. MS is now considered to be a multi-phasic disease where periods of clinical quiescence (remissions) occur between exacerbations of the disease. Remissions vary in length (typical frequency of 0.8 - 1.2/year) and may last several years but are infrequently permanent. Four courses of the disease are individualized: relapsing-remitting (RR), secondary progressive (SP), primary progressive (PP) and progressive relapsing (PR) multiple sclerosis. A total of 85 - 90% of patients experience a relapsing/remitting course, which over time becomes secondary progressive in the majority of patients; 10 - 15% have a progressive course from the onset (primary progressive MS). Over time, the relapse frequency declines.

MS is considered to be an autoimmune disorder pathologically characterized by inflammation, demyelination and variable degrees of axonal degeneration and gliosis. Demyelination of the axons, which can be partial, interferes with the conduction of action potentials along axons, which results in tissue atrophy resulting by the symptoms and neurological deficits and clinical disability experienced by patients.

The diagnosis of MS requires demonstration of dissemination of the disease process in both time and space, and exclusion of other causes. The diagnosis is made clinically with the use of paraclinical tools. The introduction of the McDonald criteria allows a diagnosis of MS to be made after a single clinical event *(*Mc Donald et al., 2001, Ann. Neurol., 50:121-127*).* MS onset is defined by the occurrence of the first neurological symptoms of CNS dysfunction. Advances in cerebrospinal fluid (CSF) analysis and magnetic resonance imaging (MRI) have simplified the diagnostic process and facilitated early diagnostic of multiple sclerosis *(*Noseworthy et al., 2000, The New England Journal of Medicihe, , 343, 13, 938-952*).* The International Panel on the Diagnosis of MS published revised criteria facilitating the diagnosis of MS and including MRI together with clinical and para-clinical diagnostic methods *(Mc Donald et al., 2001, above).*

Cell adhesion molecules (CAMs) are plasma membrane proteins specialized in cell-cell recognition and adhesion. Nectins are a group of immunoglobulin (Ig) superfamily proteins that mediate Ca2+-independent cell-cell adhesion through both homophilic as well as heterophilic interactions. The nectin family is comprised of nectin -1, -2, -3 and -4. In addition, five nectin-like proteins, also called SynCAMs (synaptic CAM), have been identified and named Necl-1 (for nectin-like 1) through Necl-5 *(*Ikeda et al., 2003, The Journal of Biological Chemistry, 278, 30, 28167-28172*;* Fuchs et al., 2006, Seminars in Cancer Biology, 16, 359-366*).* Two SynCAMs (SynCAM1, alternatively named Necl-2 and SynCAM3, alternatively named Necl-1) have been shown to serve as synaptic adhesion molecule in the CNS. SynCAM1 has been found to be expressed in the brain and various organs *(*Biederer et al., 2006, Genomics, 87, 139-150*),* while SynCAM3/ Necl-1 is mainly expressed in the nervous system *(*Kakunaga et al., 2005, J Cell Sci. 118:1267-77*).* Genomic analysis has predicted that SynCAM proteins belong to a set of four proteins: SynCAM 1, 2, 3 and 4 *(Biederer et al., 2006; above)* for which different nomenclatures have been proposed, in particular nectin-like 1 to 4 (Necl-1 to -4). Necl-3 has not been characterized, Necl-4 poorly so *(*Fukami et al., 2003, Gene 323:11-18*),* and Necl-5 is only distantly related to Necl-1 to -4.

Multiple sclerosis pathogenesis is described as a succession of immunological events. Although the exact course is not fully understood yet, it is believed that the following events are essential. T-cell activation occurs first when the receptor of the T-cells sees an antigen in the context of major histocompatibility complex (MHC) class II on the antigen-presenting cell (APC), then the activated T-cells which are initially in the periphery enter the CNS through complex mechanisms involving among other adhesion molecules. After the entry of the activated T cells into the CNS, recruitment of other immune cells occurs, a variety of cytokines are secreted, and ultimately the immune-mediated destruction of the brain parenchyma takes place. In the central nervous system, activated T cells continue to release pro-inflammatory cytokines locally, and the process repeats itself Simultaneously, other immune system cells, including B cells and macrophages, also enter the CNS through the blood-brain barrier, which further enhances the local immune response against myelin. Therefore the interaction of T-cells with adhesion molecules has been shown to be a crucial step in the pathogenesis development of multiple sclerosis.

Current medications for MS which are disease modifying treatments, i.e. modifying the course of MS, modulate or suppress the immune system. There are four immunomodulating agents approved by the Food and Drug Administration (FDA) for RRMS: three beta interferons (Betaseron®, Berlex; Avonex®, Biogen; Rebif®, Serono) and Glatiramer Acetate (Copaxone®, Amgen). There is also one FDA approved immunosuppressing drug for worsening MS, Mitoxantrone (Novantrone®, Amgen) and a humanized monoclonal antibody directed against the human α4 integrin chain, Natalizumab (Tysabri®, Biogen and Elan). Currently, the most effective MS treatment is Natalizumab but its clinical use is somewhat limited by its potential side effects. The fact that Natalizumab targets an adhesion molecule (the α4 integrin chain) exemplifies that the interaction of T-cells with adhesion molecules is crucial in the pathogenesis development of multiple sclerosis.

Due to the severity of the disease, the limited efficacy of the known treatments and the chronic character of the neurodegenerative diseases, it would be highly desirable to have a new method for treating neurodegenerative disorders, such as multiple sclerosis.

### Summary of the Invention

The present invention is directed towards new molecules capable of decreasing or altering cell adhesion and especially cell-cell contacts induced by Necl-3 and Necl-4 and their ligands, including the other members of the Necl family. More particularly, the present invention provides novel proteins and antibodies, DNA encoding thereof, processes for production thereof, pharmaceutical compositions, kits containing thereof and use of these in the preparation of pharmaceutical compositions for the treatment of CNS disorders such as neuroinflammatory and neurodegenerative conditions.

A first aspect of the invention provides a polypeptide having at least 80% identity or homology with a sequence of amino acids selected from an amino acid sequence of Necl-3 and an amino acid sequence selected from an amino acid sequence of Necl-4 or fragments thereof.

A second aspect of the invention relates to nucleic acid molecules encoding a polypeptide as defined above or fusion proteins as described below. Such nucleic acids also include vectors containing said molecules, in particular expression vectors.

A third aspect of the invention resides in a fusion protein having inhibitory activity to a Necl protein such as Necl-1 or Necl-2 or Necl-3 or Necl-4 or a ligand thereof.

A fourth aspect of the invention relates to antibodies that selectively bind the polypeptides according to the invention, as well as to antibodies that bind Necl-3 (Necl-4) and/or block the binding of Necl-3 (Necl-4) to other binding partners such as Necl-1, Necl-2, Necl-3 and/or Necl-4 and to a use of an antibody according to the invention in an assay.

A fifth aspect of the invention relates to host cells expressing a polypeptide as defined above, as well as methods of producing such cells.

A sixth aspect of the invention is a process for preparing a polypeptide or a fusion protein as defined above, typically using recombinant technologies.

A seventh aspect of the invention relates to a pharmaceutical composition comprising a polypeptide or a nucleic acid as defined above and a pharmaceutically acceptable carrier or vehicle.

An eighth aspect of the invention is a use of a Necl-3 antagonist and/or Necl-4 antagonist for the preparation of a medicament for the treatment of CNS disorders.

A ninth aspect of the invention is a Necl-3 antagonist or a Necl-4 antagonist for use as a medicament.

A tenth aspect of the invention provides a kit comprising at least one Necl-3 antagonist and/or Necl-4 antagonist according to the invention.

An eleventh aspect of the invention relates to a method of treating of a disease comprising the administration of a therapeutically effective amount of a Necl-3 antagonist or a Necl-4 antagonist according to the invention in a mammal in need thereof and wherein the disease is a CNS disorders as well as methods of promoting remyelination of nerve cells in a mammal comprising administering to the mammal a Necl-3 and/or Necl-4 antagonist according to the invention.

A twelfth aspect of the invention is a method of antagonizing the aggregation of Necl-3 and/or of Necl-4 with itself of with at least one protein of the Necl/SynCAM family.

A thirteenth aspect is a method for *in vitro* detection of the level of a Necl-3 and of Necl-4 protein in a biological sample.

A fourteenth aspect is a method for screening for a Necl-3 and/or Necl-4 antagonist.

Other features and advantages of the invention will be apparent from the following detailed description.

### Description of the figures

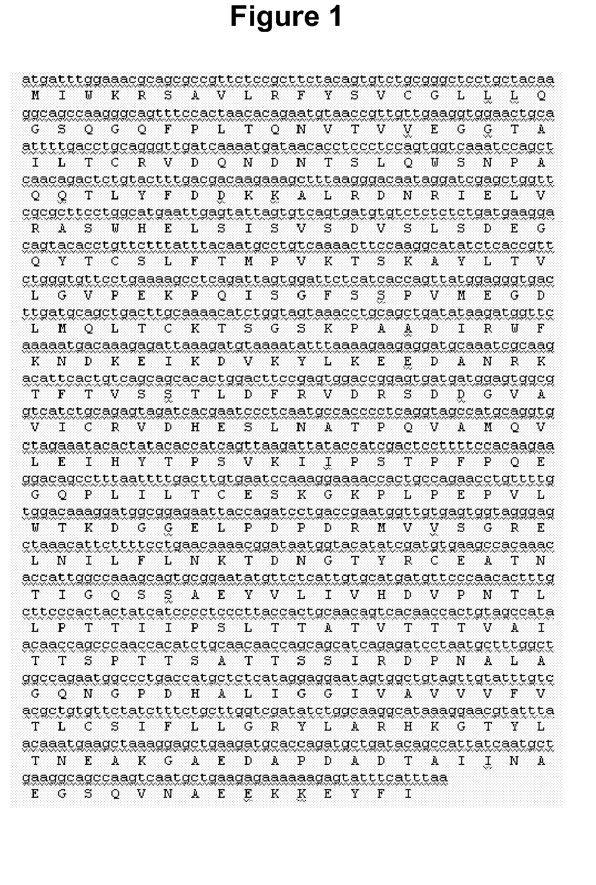

Figure 1 shows the nucleic acid sequence (SEQ ID NO: 1) and the amino acid sequence of human Necl-3 (SEQ ID NO: 2).

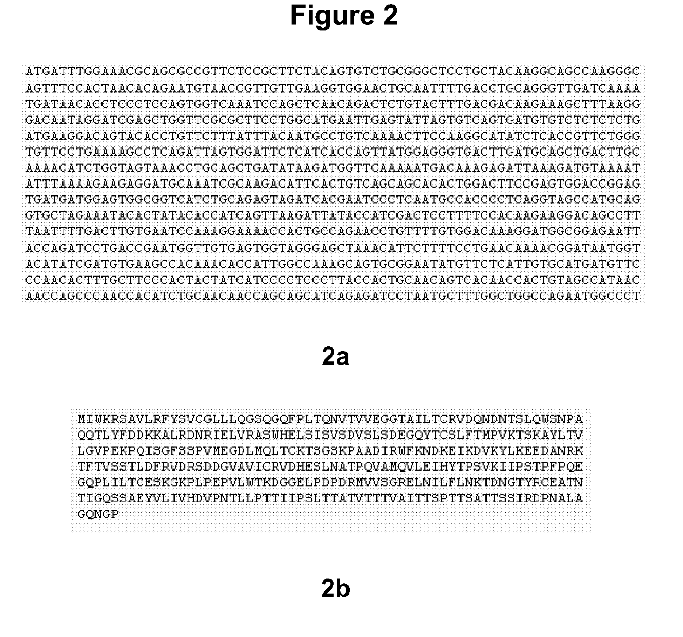

Figure 2: Figure 2a shows the nucleic acid sequence (SEQ ID NO: 3) and Figure 2b shows the amino acid sequence of the extracellular fragment aa 1-365 of human Necl-3 (SEQ ID NO: 4).

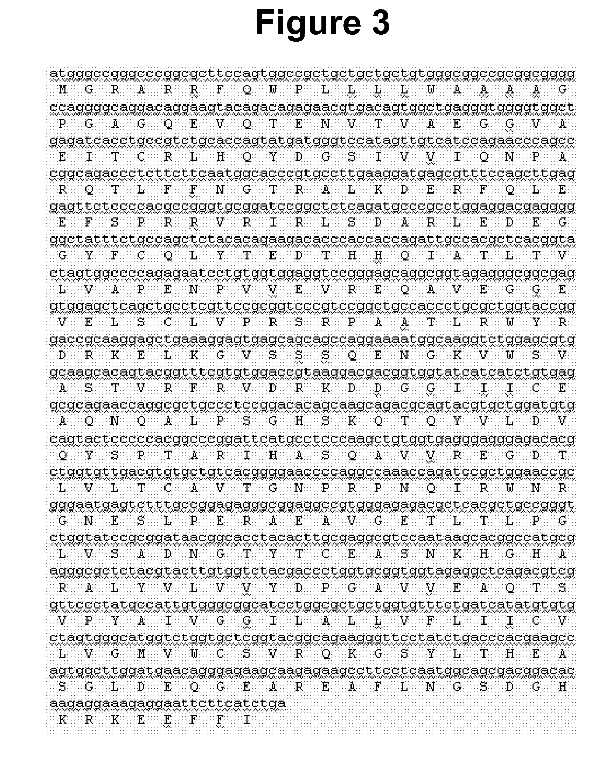

Figure 3 shows the nucleic acid sequence (SEQ ID NO: 5) and the amino acid sequence of human Necl-4 (SEQ ID NO: 6).

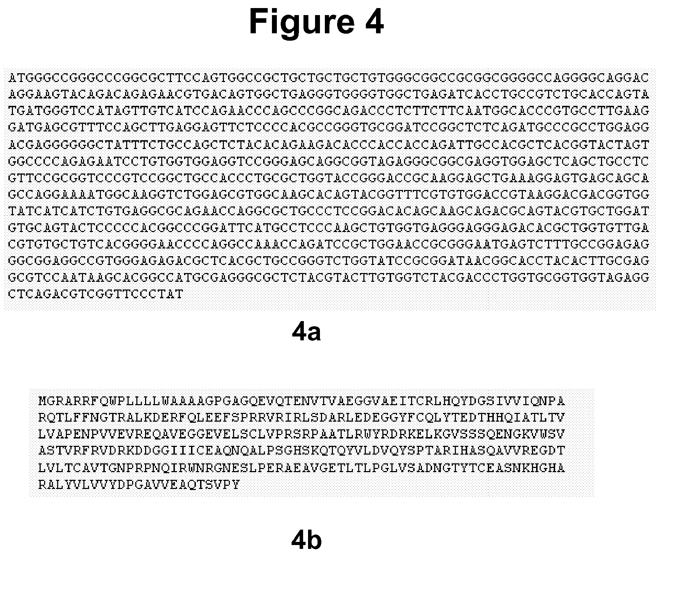

Figure 4: Figure 4a shows the nucleic acid sequence (SEQ ID NO: 7) and Figure 4b shows the amino acid sequence of the extracellular fragment aa 1-323 of human Necl-4 (SEQ ID NO: 8).

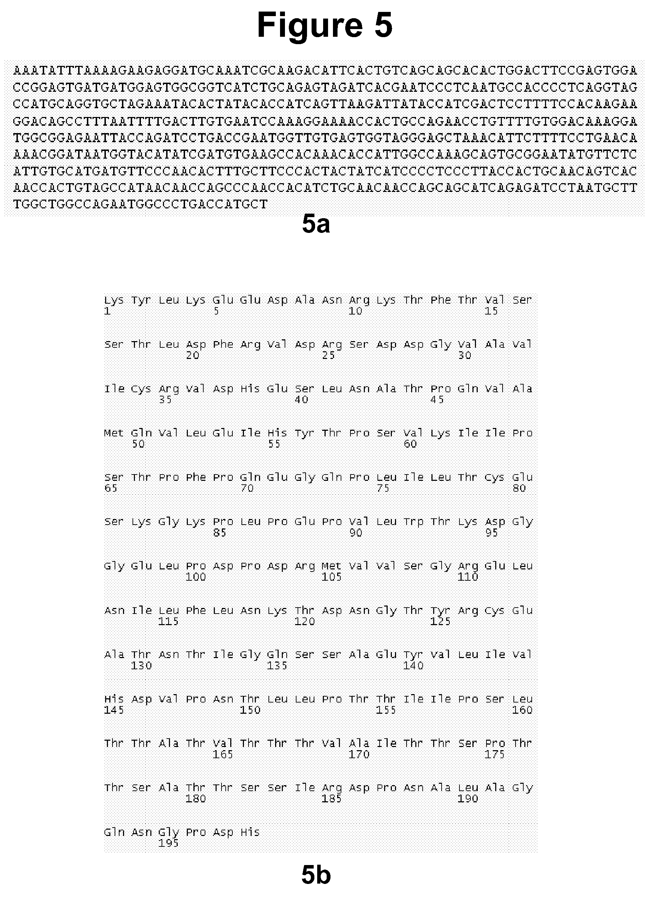

Figure 5: Figure 5a shows the nucleic acid sequence (SEQ ID NO: 14) and Figure 4b shows the amino acid sequence of the extracellular fragment aa 169-367 of human Necl-3 (SEQ ID NO: 15).

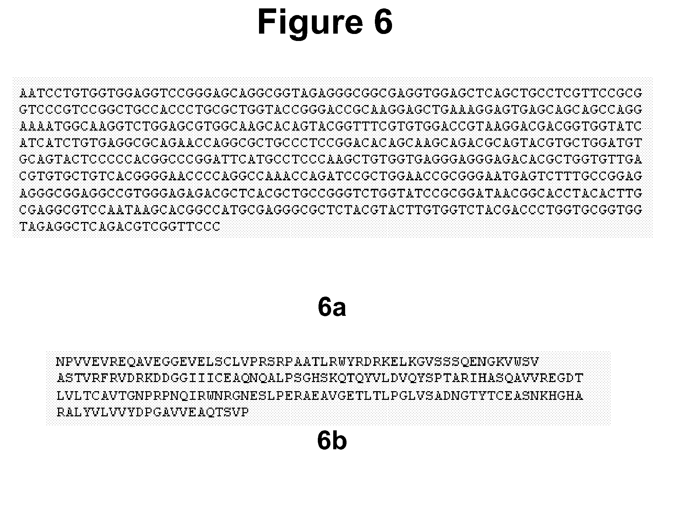

Figure 6: Figure 6a shows the nucleic acid sequence (SEQ ID NO: 25) and Figure 6b shows the amino acid sequence of the extracellular fragment aa 126-322 of human Necl-4 (SEQ ID NO: 26).

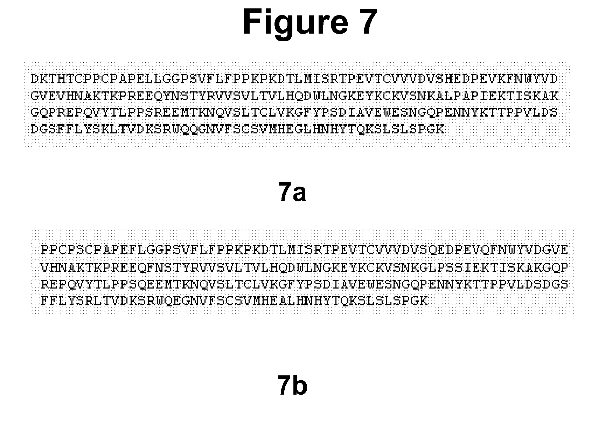

Figure 7: Figure 7a shows the amino acid sequence (SEQ ID NO: 35) of IgG1 and Figure 7b shows the amino acid sequence of IgG4 (SEQ ID NO: 36) of the fusion proteins according to the invention.

### Detailed Description of the invention

The term "neurodegenerative" comprises a disease or a state characterized by a CNS degeneration or alteration, especially at the level of the neurons. It comprises neuro-inflammatory and demyelinating states or diseases such as leukoencephalopathies, and leukodystrophies.

The term "demyelinating" is referring to a state or a disease of the CNS comprising the degradation of the myelin around the axons. In the context of the invention, the term demyelinating disease is intended to comprise conditions which comprise a process that demyelinate cells such as multiple sclerosis, progressive multifocal leukoencephalopathy (PML), myelopathies, any neuroinflammatory condition involving autoreactive leukocyte within the CNS, congenital metabolic disorder, a neuropathy with abnormal myelination, drug induced demyelination, radiation induced demyelination, a hereditary demyelinating condition, a prion induced demyelinating condition, encephalitis induced demyelination or a spinal cord injury. Preferably, the condition is multiple sclerosis.

The term "multiple sclerosis" includes relapsing remitting MS (RRMS), Secondary Progressive MS (SPMS), primary progressive MS (PPMS). Patients suffering from MS can be defined for example as having clinically definite or laboratory-definite MS according to Schumacher or Poser criteria *(*Schumacher et al., 1965, Ann. NYAcad. Sci. 1965; 122:552-568*;* Poser et al., 1983, Ann. Neurol. 13(3): 227-31*).*

The term "Efficacy" of a treatment according to the invention can be measured based on changes in the course of disease in response to a use according to the invention. For example, the efficacy of a treatment of MS can be measured by the frequency of relapses in RRMS and the presence or absence of new lesions in the CNS as detected using methods such as MRI technique *(*Miller et al., 1996, Neurology, 47(Suppl 4): S217*;* Evans et al., 1997, Ann. Neurology, 41:125-132*).* The observation of the reduction and/or suppression of MRI T₁ gadolinium-enhanced lesions (thought to represent areas of active inflammation) gives a primary efficacy variable. Secondary efficacy variables include MRI T₁ enhanced brain lesion volume, MRI T₁ enhanced lesion number, MRI T₂ lesion volume (thought to represent total disease burden, i.e. demyelination, gliosis, inflammation and axon loss), MRI T₁ enhanced hypointense lesion volume (thought to represent primarily demyelination and axon loss), time-to-progression of MS, frequency and severity of exacerbations and time-to-exacerbation, Expanded Disability Status Scale score and Scripps Neurologic Rating Scale (SNRS) score *(*Sipe et al., 1984, Neurology, 34, 1368-1372*).* Methods of early and accurate diagnosis of multiple sclerosis and of following the disease progression are described in Mattson, 2002, Expert Rev. Neurotherapeutics, 319-328*.* Degree of disability of MS patients can be for example measured by Kurtzke Expanded Disability Status Scale (EDSS) score *(*Kurtzke, 1983, Neurology, 33, 1444-1452*).* Typically a decrease in EDSS score corresponds to an improvement in the disease and conversely, an increase in EDSS score corresponds to a worsening of the disease. Any other clinical assessment of the disease known is available to the skilled person to measure the efficacy of the treatment according to the invention.

"Relapses" involve neurological problems that occur over a short period, typically days but sometimes as short as hours or even minutes. These attacks most often involve motor, sensory, visual or coordination problems early in the disease. Later, bladder, bowel, sexual and cognitive problems may be shown. Sometimes the attack onset occurs over several weeks. Typical MS relapse involves a period of worsening, with development of neurological deficits, then a plateau, in which the patient is not getting any better but also not getting any worse followed by a recovery period. Recovery usually begins within a few weeks.

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e., arresting its development; or relieving the disease, i.e., causing regression of the disease and/or its symptoms or conditions.

The term "subject" as used herein refers to mammals. For examples, mammals contemplated by the present invention include human, primates, domesticated animals such as cattle, sheep, pigs, horses and the like.

The term "Fc domain" encompasses native Fc, including the various subtypes, and Fc variant molecules and sequences as defined below. It includes molecules in monomeric or multimeric form, whether digested from whole antibody or produced by other means.

The term "native Fc" refers to a molecule or sequence comprising the sequence of a non-antigen binding fragment resulting from the digestion of whole antibody, or by means of recombinant DNA techniques, whether in monomeric or multimeric form. The original immunoglobulin source of native Fc is preferably of human origin and may be any of the immunoglobulins. Native Fc's are made up of monomeric polypeptides that may be linked into dimeric or multimeric forms by covalent (i.e. disulfide bonds) and non-covalent association. The number of intermolecular disulfide bonds between monomeric subunits of native Fc molecules ranges from 1 to 4 depending on class (e. g. IgG, IgA, IgE, IgM, IgD) or subclass (e.g. IgG1, IgG2, IgG3, IgG4, IgAl, IgA2). Examples of native Fc domains are human IgG1 and IgG4, having respectively the amino acid sequence of SEQ ID NO: 35 and of SEQ ID NO: 36. The term "native Fc" as used in the context of the invention is generic to the monomeric, dimeric, and multimeric forms.

The term "Fc variant" refers to a molecule or sequence that is modified from a native Fc, for example a molecule or sequence that is humanized from a non-human native Fc. A native Fc comprises sites that may be removed or changed because they provide structural features or biological activities that are not required, or alternatively enhanced, for the fusion molecules of the present invention. Thus, the term "Fc variant" comprises a molecule or sequence that lacks one or more native Fc sites or residues that affect or are involved in (i) disulfide bond formation, (ii) incompatibility with a selected host cell (iii) N-terminal heterogeneity upon expression in a selected host cell, (iv) glycosylation, (v) interaction with complement, (vi) binding to an Fc receptor other than a salvage receptor, or (vii) antibody-dependent cellular cytotoxicity (ADCC).

The term "Fc fusion proteins" in general refers to molecules comprising at least part of an immunoglobulin Fc domain and at least one peptide. Such fusion proteins may be multimers or dimers or fragment thereof and may be derivatized. Suitable fusion proteins of the invention include a Necl-3 polypeptide e.g. an extracellular domain of Necl-3, a fragment thereof or a mutein thereof, linked to an immunoglobulin Fc region.

Suitable fusion proteins of the invention include a Necl-4 polypeptide e.g. an extracellular domain of Necl-4, a fragment thereof or a mutein thereof, linked to an immunoglobulin Fc region. The extracellular domain, the fragment or the mutein thereof may be fused directly or through linker sequences to the Fc portion of an immunoglobulin. The preparation of fusion proteins comprising certain heterologous polypeptides fused to an Fc domain is known in the art WO 91/08298, WO 96/08570, WO 93/22332, WO 04/085478, WO 01/03737 and WO 02/66514 incorporated by reference in their entirety.

The term "isolated" is used to indicate that the molecule is free of association with other proteins or polypeptides, for example as a purification product of recombinant host cell culture or as a purified extract.

The term "antibody" comprises antibodies binding to Necl-3 or Necl-4 protein or fragment thereof, chimeric antibodies recognizing and/or binding selectively to Necl-3 or Necl-4 protein or fragment thereof, fully human, humanized, genetically engineered or bispecific or multispecific antibodies as well as fragments thereof such as single chain antibodies (scFv) or domain antibodies against Necl-3 and/or Necl-4 protein or fragment thereof and the like. Antibodies of this invention may be monoclonal or polyclonal antibodies, or fragments or derivative thereof having substantially the same antigen specificity. The term "selectively" indicates that the antibodies preferentially recognize and/or bind the target polypeptide or epitope, i.e., with a higher affinity than any binding to any other antigen or epitope, i.e. the binding to the target polypeptide can be discriminated from non-specific binding to other antigens. The binding affinity of an antibody can be readily determined by one of ordinary skill in the art, for example, by Scatchard analysis *(*Scatchard et al., 1949, Ann NY Acad. Sci., 51, 660-672*).*

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. The modifier "monoclonal" indicates the character of the antibody as being obtained from a substantially homogeneous population of antibodies, and is not to be construed as requiring production of the antibody by any particular method.

The term "antagonists" is defined as a molecule that antagonizes completely or partially the activity of biological molecule.

The term "peptidomimetic" is defined as a peptide analog containing non-peptidic structural elements, which peptide is capable of mimicking or antagonizing the biological action(s) of a natural parent peptide. A peptidomimetic does no longer have classical peptide characteristics such as enzymatically scissile peptide bonds.

### Necl-3 and Necl-4

The nucleotide sequence encoding human Necl-3, isolated as described in Examples 1 and 2, is presented in SEQ ID NO: 1 and the amino acid sequence of human Necl-3 is described in SEQ ID NO: 2. The human Necl-3 amino acid sequence described in SEQ ID NO: 2 has a predicted extracellular domain spanning from amino acids 1 to 367.

As used herein, the term Necl-3 encompasses polypeptides having an amino acid sequence of SEQ ID NO: 2 and fragments thereof such as the corresponding full length extracelullar domain (aa1-365) of SEQ ID NO: 4 or a fragment thereof (aa169-367) having an amino acid sequence of SEQ ID NO: 15. In addition, Necl-3 encompasses polypeptides that have a high degree of similarity or a high degree of identity with the amino acid sequence of SEQ ID NO: 2, or with the amino acid sequence of SEQ ID NO: 4 or the amino acid sequence of SEQ ID NO: 15 and which polypeptides are biologically active. The term Necl-3 encompasses Necl-3 secreted peptide where the signal peptide is cleaved. Typically, the signal peptide is cleaved between position 24 and 25 of the SEQ ID NO: 4 as determined by Signal P 3.0 software.

The nucleotide sequence encoding human Necl-4, isolated as described in Examples 6 and 7, is presented in SEQ ID NO: 5 and the amino acid sequence of human Necl-4 is described in SEQ ID NO: 6. The human Necl-4 amino acid sequence described in SEQ ID NO: 6 has a predicted extracellular domain of from amino acids 1 to 323.

As used herein, the term Necl-4 encompasses polypeptides having an amino acid sequence of SEQ ID NO: 6 and fragments thereof such as the full length extracelullar domain (aal-323) of SEQ ID NO: 8 or a fragment thereof (aa126-322) having an amino acid sequence of SEQ ID NO: 26. In addition, Necl-4 encompasses polypeptides that have a high degree of similarity or a high degree of identity with the amino acid sequence of SEQ ID NO: 6 or with the amino acid sequence of SEQ ID NO: 8 or with the amino acid sequence of SEQ ID NO: 26 and which polypeptides are biologically active.

The term Necl-4 encompasses Necl-4 secreted peptide where the signal peptide is cleaved. Typically, the signal peptide is cleaved between position 24 and 25 of the SEQ ID NO: 8 as determined by Signal P 3.0 software.

A "Necl-3 variant" or a "Necl-4 variant" as referred to herein, means a polypeptide substantially homologous to native Necl-3 or native Nelc-4, respectively, but which has an amino acid sequence different from that of native Necl-3 or Necl-4 respectively (human, murine or other mammalian species) because of one or more deletions, insertions or substitutions. Substantially homologous means a variant amino acid sequence that is at least 80%, at least 85%, at least 90%, at least 95%, at least 96%, at least 97%, at least 98% or at least 99% identical to the native amino acid sequences, as disclosed above. The percent identity of two amino acid or two nucleic acid sequences can be determined by visual inspection and/or mathematical calculation, or more easily by comparing sequence information using a computer program such as Clustal package version 1.83. Necl-3 variants or Necl-4 variants may comprise a sequence having at least one conservatively substituted amino acid, meaning that a given amino acid residue is replaced by a residue having similar physiochemical characteristics. Generally, substitutions for one or more amino acids present in the native polypeptide should be made conservatively. Examples of conservative substitutions include substitution of amino acids outside of the active domain(s), and substitution of amino acids that do not alter the secondary and/or tertiary structure of Necl-3 or Necl-4 respectively. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known *(*Kyte, et al., 1982, J. Mol. Biol., 157: 105- 131*).* Naturally occurring variants are also encompassed by the invention. Examples of such variants are proteins that result from alternate mRNA splicing events or from proteolytic cleavage of the native protein, wherein the native biological property is retained. For example, a "conservative amino acid substitution" may involve a substitution of a native amino acid residue with a non native residue such that there is little or no effect on the polarity or charge of the amino acid residue at that position. Desired amino acid substitutions (whether conservative or non-conservative) can be determined by those skilled in the art at the time such substitutions are desired. Exemplary amino acid substitutions are presented in Table 1 below:

**Table 1**

| Original residues | Examples of substitutions |
|---|---|
| Ala (A) | Val, Leu, Ile |
| Arg (R) | Lys, Gln, Asn |
| Asn (N) | Gln |
| Asp (D) | Glu |
| Cys (C) | Ser, Ala |
| Gln (Q) | Asn |
| Glu (E) | Asp |
| Gly (G) | Pro, Ala |
| His (H) | Asn, Gln, Lys, Arg |
| Ile (I) | Leu, val, Met, Ala, Phe, Norleucine |
| Leu (L) | Ile, Val, Met, Ala, Phe, Norleucine |
| Lys (K) | Arg, Gln, Asn |
| Met (M) | Leu, Ile, Phe |
| Phe (F) | Leu, Val, Ile, Ala, Tyr |
| Pro (P) | Ala, Gly |
| Ser (S) | Thr, Ala, Cys |
| Thr (T) | Ser |
| Trp (W) | Tyr, Phe |
| Tyr (Y) | Trp, Phe, Thr, Ser |
| Val (V) | Ile, Met, Leu, Phe, Ala, Norleucine |

Further examples ofNecl-3 or Necl-4 are provided herein and include but is not limited to variants, biologically active fragments, fragments and fragments of variants. Necl-3 variants or Necl-4 variants may be used as antagonists of Necl-3 activity or Necl-4 activity or be used to develop Necl-3 antagonists or Necl-4 antagonists, such as antibodies, chimaeric proteins or fusion proteins according to the invention.

The term "Necl-3 antagonist" comprises all antagonists of all suitable forms of Necl-3 described herein that antagonize one or more biological activity of Necl-3 and of Necl-3 variants or fragment thereof. For example, the Necl-3 antagonists of the invention are able to antagonize the ability of Necl-3 to participate to homophilic interactions (interactions between Necl-3 proteins) and/or heterophilic interactions (interactions with other protein(s) from the Necl or synCAM family such as Necl-1, Necl-2 and Necl-4, or with other membrane protein ligands). The term "Necl-3 antagonist" includes but is not limited to: Necl-3 specific antibodies of any sort (polyclonal, monoclonal, antibody fragments, antibody variants), chimaeric proteins, natural or unnatural proteins with Necl-3 antagonizing activities, small molecules, nucleic acid derived polymers (such as DNA and RNA aptamers, PNAs, or LNAs), peptidomimetics, fusion proteins, or gene therapy vectors driving the expression of such Necl-3 antagonists. Further embodiments include as Necl-3 antagonists, soluble Necl-3 fusion proteins such as but not limited to an extracellular domain of Necl-3 or a fragment thereof linked to an Fc domain. Typically, Necl-3 antagonists are able to bind Necl-3 and/or to block the binding of Necl-3 and other binding partners such as Necl-1, Necl-2, Necl-3 and/or Necl-4.

The term "Necl-4 antagonist" comprises all antagonists of all suitable forms of Necl-4 described herein that antagonize one or more biological activity of Necl-4 and of Necl-4 variants. For example, the Necl-4 antagonists of the invention are able to antagonize the ability of Necl-4 to participate to homophilic interactions (interactions between Necl-4 proteins) and/or heterophilic interactions (interactions with other protein(s) from the SynCAM family such as Necl-1, Necl-2 and Necl-3, or with other membrane protein ligands). The term Necl-4 antagonist includes but is not limited to: Necl-4 specific antibodies, chimeric proteins, small molecules, peptidomimetics and fusion proteins. Further embodiments include as Necl-4 antagonists, soluble Necl-4 fusion proteins such as but not limited to an extracellular domain of Necl-4 or a fragment thereof linked to an Fc domain. Typically, Necl-4 antagonists are able to bind Necl-3 and/or to block the binding of Necl-4 and other binding partners such as Necl-1, Necl-2, Necl-3 and/or Necl-4.

The biological consequences of the homophilic and heterophilic interactions referred in the context of the invention for instance lead to cell-cell contact, to cell movement, to cell aggregation or to cellular activation.

An antibody that is immunoreactive with Necl-3 and/or especially to an extracellular fragment of Necl-3 or a fragment thereof e.g. to an amino acid sequence of SEQ ID NO: 4 or SEQ ID NO: 15 may be used as a Necl-3 antagonist.

An antibody that is immunoreactive with Necl-4 and/or especially to an extracellular fragment of Necl-4 or a fragment thereof e.g. to an amino acid sequence of SEQ ID NO: 8 or SEQ ID NO: 26 may be used as a Necl-4 antagonist.

A Necl-3 or a Necl-4 protein, a Necl-3 antagonist or a Necl-4 antagonist, as an isolated, purified or homogeneous protein according to the invention, may be produced by recombinant expression systems as described herein or purified from naturally occurring cells.

Suitable expression of Necl-3, Necl-3 variants or fragments, Necl-3 antagonists, Necl-4, Necl-4 variants or fragments and Necl-4 include prokaryotes, yeast or higher eukaryotic cells. Appropriate cloning and expression vectors for use with bacterial, fungal, yeast and mammalian cellular hosts are described for example in Pouwels et al., 1985, Cloning Vectors: A laboratory manual, Elsevier New York*.*

Prokaryotes include gram negative and gram positive organism such as *E. Coli* or *Bacilli.* Suitable prokaryotic host cells include for example *E. Coli BL21* strain. In prokaryotic host cells, such as *E. coli,* a Necl-3, Necl-3 fragment or variant, Necl-3 antagonist, Necl-4, Necl-4 fragment or variant or Necl-4 antagonist may include a N-terminal methionine residue to facilitate the expression of recombinant polypeptide in the prokaryotic host cell. The N-terminal Met may be cleaved from the expressed peptide.

### Compositions

The invention provides pharmaceutical or therapeutic agents as compositions and methods for treating a patient, preferably a mammalian patient, and most preferably a human patient who is suffering from a medical disorder, and in particular a disorder mediated by Necl-3 and/or Necl-4, such as neuroinflammatory disorders and/or neurodegenerative disorders.

Pharmaceutical compositions of the invention can contain one or more Necl-3 antagonist and/or Necl-4 antagonist (including from recombinant and non-recombinant sources) in any form described herein. Compositions of this invention may further comprise one or more pharmaceutically acceptable additional ingredient(s) such as alum, stabilizers, antimicrobial agents, buffers, coloring agents, flavoring agents, adjuvants, and the like.

The compounds of the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed. Compositions according to the invention are preferably injectable.

Compositions of this invention may also be liquid formulations including, but not limited to, aqueous or oily suspensions, solutions, emulsions, syrups, and elixirs. Liquid forms suitable for oral administration may include a suitable aqueous or non-aqueous vehicle with buffers, suspending and dispensing agents, colorants, flavors and the like. The compositions may also be formulated as a dry product for reconstitution with water or other suitable vehicle before use. Such liquid preparations may contain additives including, but not limited to, suspending agents, emulsifying agents, non-aqueous vehicles and preservatives. Suspending agent include, but are not limited to, sorbitol syrup, methyl cellulose, glucose/sugar syrup, gelatin, hydroxyethylcellulose, carboxymethyl cellulose, aluminum stearate gel, and hydrogenated edible fats. Emulsifying agents include, but are not limited to, lecithin, sorbitan monooleate, and acacia. Nonaqueous vehicles include, but are not limited to, edible oils, almond oil, fractionated coconut oil, oily esters, propylene glycol, and ethyl alcohol. Preservatives include, but are not limited to, methyl or propyl p-hydroxybenzoate and sorbic acid. Further materials as well as processing techniques and the like are set out in Part 5 of Remihgtoh's Pharmaceutical Sciences, 20th Edition, 2000, Marck Publishing Company, Easton, Pennsylvania, which is incorporated herein by reference.

Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate. Tablets may be coated according to methods well known in the art.

Injectable compositions are typically based upon injectable sterile saline or phosphate-buffered saline or other injectable carriers known in the art.

Compositions of this invention may also be formulated as suppositories, which may contain suppository bases including, but not limited to, cocoa butter or glycerides. Compositions of this invention may also be formulated for inhalation, which may be in a form including, but not limited to, a solution, suspension, or emulsion that may be administered as a dry powder or in the form of an aerosol using a propellant, such as dichlorodifluoromethane or trichlorofluoromethane. Compositions of this invention may also be formulated transdermal formulations comprising aqueous or non-aqueous vehicles including, but not limited to, creams, ointments, lotions, pastes, medicated plaster, patch, or membrane.

Compositions of this invention may also be formulated for parenteral administration including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.

Compositions of this invention may also be formulated as a depot preparation, which may be administered by implantation or by intramuscular injection. The compositions may be formulated with suitable polymeric or hydrophobic materials (as an emulsion in an acceptable oil, for example), ion exchange resins, or as sparingly soluble derivatives (as a sparingly soluble salt, for example).

Compositions of this invention may also be formulated as a liposome preparation. The liposome preparation can comprise liposomes which penetrate the cells of interest or the *stratum corneum,* and fuse with the cell membrane, resulting in delivery of the contents of the liposome into the cell. Other suitable formulations can employ niosomes. Niosomes are lipid vesicles similar to liposomes, with membranes consisting largely of non-ionic lipids, some forms of which are effective for transporting compounds across the stratum corneum.

The compounds of this invention can also be administered in sustained release forms or from sustained release drug delivery systems. A description of representative sustained release materials can also be found in the incorporated materials in *Remington's Pharmaceutical Sciences.*

### Mode of administration

Compositions of this invention may be administered in any manner including, but not limited to, orally, parenterally, sublingually, transdermally, rectally, transmucosally, topically, via inhalation, via buccal or intranasal administration, or combinations thereof Parenteral administration includes, but is not limited to, intravenous, intra-arterial, intraperitoneal, subcutaneous, intramuscular, intra-thecal, and intra-articular. The compositions of this invention may also be administered in the form of an implant, which allows slow release of the compositions as well as a slow controlled i.v. infusion. In a preferred embodiment, a Necl-3, or a Necl-3 antagonist, or a necl-4 or a Necl-4 antagonist according to the invention are administered intravenously or subcutaneously.

This invention is further illustrated by the following examples that are not intended to limit the scope of the invention in any way.

The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

### Combination

According to the invention, a Necl-3 antagonist and/or a Necl-4 antagonist according to the invention can be administered alone or in combination with a co-agent useful in the treatment of CNS disorders including neuroinflammation and/or neurodegeneration, such as substances useful in the treatment of demyelinating diseases e.g. for example a co-agent selected from IFN-beta, Glatiramer acetate, Corticosteroids, immunusuppressants such as Mitoxantrone, Azathioprine, Cyclophosphamide, Methotrexate, Natalizumab (Tysabri®), metalloproteinases inhibitors, especially MMP-12 and/or MMP-9 inhibitors such as Minocycline.

The invention encompasses the administration of a Necl-3 antagonist or Necl-4 antagonist of the invention wherein the Necl-3 antagonist or the Necl-4 antagonist is administered to an individual prior to, simultaneously or sequentially with other therapeutic regimens or co-agents useful in the treatment of neuroinflammation and/or neurodegeneration (e.g. multiple drug regimens), in a therapeutically effective amount. The Necl-3 antagonist and/or a Necl-4 antagonist according to the invention that are administered simultaneously with said co-agents can be administered in the same or different compositions and in the same or different routes of administration.

The term "interferon-beta (IFN-β)", as used herein, is intended to include fibroblast interferon in particular of human origin, as obtained by isolation from biological fluids or as obtained by DNA recombinant techniques from prokaryotic or eukaryotic host cells, as well as its salts, functional derivatives, variants, analogs and active fragments.

IFN-β suitable in accordance with the present invention is commercially available e.g. as Rebif® (Serono), Avonex® (Biogen) or Betaferon® (Schering). The use of interferons of human origin is also preferred in accordance with the present invention. The term interferon, as used herein, is intended to encompass salts, functional derivatives, variants, analogs and active fragments thereof

### Patients

In an embodiment, patients according to the invention are patients suffering from CNS disorders such as neuroinflammatory and neurodegenerative disorders or states.

In another embodiment, patients according to the invention are patients suffering from demyelinating states or disorders.

In a further embodiment, patients according to the invention are patients suffering from multiple sclerosis.

### Use according to the invention

The nucleic acids encoding Necl-3, Necl-3 antagonists, Necl-4, Necl-4 antagonists and fragments thereof may be used to express recombinant polypeptides for analysis, characterization and therapeutic use.

Necl-3 antagonists and Necl-4 antagonists according to the invention are useful in the treatment of CNS disorders, including neuroinflammatory and/or neurodegenerative disorders or conditions such as demyelinating diseases or conditions.

Necl-3 antagonists according to the invention are useful to antagonize one or more biological activity of Necl-3 such as adhesion properties, including Necl-3 induced cell adhesion processes, for example adhesion process between Necl-3 on myelinated axons and cells of the immune system expressing Necl-3 or Necl-3 heterophilic ligands.

Necl-4 antagonists according to the invention are useful to antagonize one or more biological activity of Necl-4 such as adhesion properties, including Necl-4 induced cell adhesion processes, for example adhesion process between Necl-4 on astrocytes and cells of the immune system expressing Necl-4 or Necl-4 heterophilic ligands

The disclosed Necl-3 nucleic acid sequences or Necl-4 nucleic acid sequences, or fragments thereof and combinations of fragment thereof may be used as probes or primers.

The disclosed Necl-3 or Necl-4 amino acid sequences, variants thereof, fragments thereof and combinations thereof may be used in a process for the preparation of Necl-3 antagonists and/or Necl-4 antagonists according to the invention.

The disclosed Necl-3 antibodies and/or Necl-4 antibodies may be used in assays relating for example to the analysis of Necl-3 and/or Necl-4 expression such as western blots, immunohistochemistry, ELISA or FACS assays.

Within the context of this invention, the beneficial effect includes but is not limited to an attenuation, reduction, decrease or diminishing of the pathological development after onset of the disease.

Table 2 below presents the Sequence identity numbers and associated molecules:

**Table 2**

| SEQ ID NO. | Molecule |
|---|---|
| 1 | DNA sequence for human Necl-3 |
| 2 | Amino acid sequence for human Necl-3 |
| 3 | DNA sequence for full extracellular domain of human Necl-3 (1-365) |
| 4 | Amino acid sequence for full extracellular domain of human Necl-3 (1-365) |
| 5 | DNA sequence for human Necl-4 |
| 6 | Amino acid sequence for human Necl-4 |
| 7 | DNA sequence for full extracellular domain of human Necl-4 (1-323) |
| 8 | Amino acid sequence for full extracellular domain of human Necl-4 (1-323) |
| 9 | cDNA of clone DKFZp761G128 |
| 10 | PCR primer for Necl-3 construct |
| 11 | PCR primer for Necl-3 construct |
| 12 | PCR primer for Necl-3 fragment (169-367) and (1-365) constructs |
| 13 | PCR primer for Necl-3 fragment (169-367) and (1-365)constructs |
| 14 | Nucleic acid sequence for a Necl-3 fragment (169-367) |
| 15 | Amino acid sequence for a Necl-3 fragment (169-367) |
| 16 | RT PCR primer for Necl-3 |
| 17 | RT PCR primer for Necl-3 |
| 18 | RT PCR primer for GAPDH |
| 19 | RT PCR primer for GAPDH |
| 20 | Modified cDNA of clone IMAGE 4375136 |
| 21 | PCR primer for Necl-4 construct |
| 22 | PCR primer for Necl-4 construct |
| 23 | PCR primer for Necl-4 fragment (126-322) construct |
| 24 | PCR primer for Necl-4 fragment (126-322) construct |
| 25 | Nucleic acid sequence for a Necl-4 fragment (126-322) |
| 26 | Amino acid sequence for a Necl-4 fragment (126-322) |
| 27 | RT PCR primer for Necl-4 |
| 28 | RT PCR primer for Necl-4 |
| 29 | cDNA of clone IMAGE:5199627 |
| 30 | PCR primer for Necl-1 construct |
| 31 | PCR primer for Necl-1 construct |
| 32 | cDNA of clone IMAGE:4701395 |
| 33 | PCR primer for Necl-2 construct |
| 34 | PCR primer for Necl-2 construct |
| 35 | Amino acid sequence for Human IgG1 |
| 36 | Amino acid sequence for Human IgG4 |
| 37 | Amino acid sequence for Mouse IgG2a |
| 38 | Amino acid sequence for Mouse IgG3 |
| 39 | Amino acid sequence for Necl-1 |
| 40 | Amino acid sequence for Necl-2 |
| 41 | Amino acid sequence for MOG peptide |

One process for producing Necl-3 antagonists or Necl-4 antagonists according to the invention comprises culturing a host cell transformed with an expression vector comprising a DNA sequence that encodes a Necl-3 antagonist or a Necl-4 antagonist under conditions sufficient to promote expression of Necl-3 antagonists or Necl-4 antagonists, respectively.

A Necl-3 antagonist or a Necl-4 antagonist according to the invention is then recovered from culture medium or cell extracts, depending upon the expression system employed. As known to the skilled artisan, procedures for purifying a recombinant protein will vary according to such factors as the type of host cells employed and whether or not the recombinant protein is secreted into the culture medium.

For example, when expression systems that secrete the recombinant protein are employed, the culture medium first may be concentrated using a commercially available protein concentration filter, for example, an Amicon or Millipore Pellicon ultrafiltration unit.

Following the concentration step, the concentrate can be applied to a purification matrix such as a gel filtration medium. Alternatively, an anion exchange and/or an affinity resin can be employed. The matrices can be acrylamide, agarose, dextran, cellulose or other types commonly employed in protein purification. Alternatively, a cation exchange step can be employed. Finally, one or more reversed-phase high performance liquid chromatography (RP-HPLC) steps employing hydrophobic RP-HPLC media can be employed to further purify Necl-3, Necl-3 variants and Necl-3 antagonists and/or Necl-4, Necl-4 variants and Necl-4 antagonists. Some or all of the foregoing purification steps, in various combinations, are well known and can be employed to provide a substantially homogeneous recombinant protein.

Recombinant protein produced in bacterial culture can be isolated by initial disruption of the host cells, centrifugation, extraction from cell pellets if an insoluble polypeptide, or from the supernatant fluid if a soluble polypeptide, followed by one or more concentration, salting-out, ion exchange, affinity purification or size exclusion chromatography steps.

Microbial cells can be disrupted by any convenient method, including freeze-thaw cycling, sonication, mechanical disruption, or use of cell lysing agents.

A desired DNA sequence may be chemically synthesized using techniques known per se. DNA fragments also may be produced by restriction endonuclease digestion of a full length cloned DNA sequence, and isolated by electrophoresis on agarose gels. Linkers containing restriction endonuclease cleavage site(s) may be employed to insert the desired DNA fragment into an expression vector, or the fragment may be digested at cleavage sites naturally present therein. The well known polymerase chain reaction procedure also may be employed to amplify a DNA sequence encoding a desired protein fragment. As a further alternative, known mutagenesis techniques may be employed to insert a stop codon at a desired point, e. g. immediately downstream of the codon for the last amino acid of the receptor-binding domain.

Typically, when the Necl-3 and/or Necl-4 antagonists are fusion proteins (Fc-Necl-3 and Fc-Necl-4 fusion protein) according to the invention, they may be prepared according to known processes: for example, the nucleic acid sequence encoding the extracellular domain of Necl-3 or Necl-4 or a fragment thereof can be cloned in an expression vector fused to a nucleic acid sequence encoding the original extracellular domain of Necl-3 or Necl-4 or a fragment thereof signal sequence (or any other appropriate signal/export sequence) at its 5' end, and the nucleic acid sequence encoding the constant region of human immunoglobulin lambda heavy chain IgG (e.g. IgG1 or IgG4) at its 3'end. The resulting vector can be used to transform a mammalian (e.g. CHO or HEK293) or an insect (e.g. Schneider S2 cells) host cell line and the clones stably expressing and secreting the recombinant fusion protein having extracellular domain of Necl-3 or Necl-4 or a fragment thereof at the N-terminus and the IgG sequence at the C-terminus can be selected. This clone then can be used for scaling up the production and for purifying the recombinant fusion protein from the culture medium. Alternatively, the position of the nucleic acid encoding the constant region of human immunoglobulin lambda heavy chain IgG and extracellular domain of Necl-3 or Necl-4 or a fragment thereof can be inversed, and the resulting protein can be expressed and secreted using still the original signal sequence of extracellular domain of Necl-3 or Necl-4 or a fragment thereof, or any other appropriate signal/export sequence. Using these technologies it can be also possible to generate heterodimers if two different constructs expressing one Necl-3-Fc or Necl-4-Fc fusion protein and the other a different Fc-based fusion protein (for example another antagonist) are coexpressed in the same host cell (WO 00/18932).

Typically, preparation of the fusion protein according to the invention in insect cells is carried out by the co-transfection of the corresponding insect cell expression vector expressing Fc-Necl-3 extra-cellular domain or Fc-Necl-4 extra-cellular domain (such as illustrated in Examples 6 and 12) along with the plasmid pCoBlast that carries a blasticidin S resistance gene and stable clones are selected with blasticidin S according to methods commonly known in the art. Of these, high expressers of the fusion proteins are selected.

Alternatively, the preparation in mammalian cells (CHO, BHK, COS, HEK-293 or other cell line) is carried out by the transfected of the corresponding mammalian cell expression vector expressing Fc-Necl-3 extra-cellular domain or Fc-Necl-4 (such as illustrated in Examples 6 and 12) and stable clones are selected with Zeocin according to methods commonly known in the art. Of these, high expressers of the fusion proteins are selected.

Once selected, high expresser clones obtained from one of these methods are expanded and grown (e.g. in a 150 mL flat bottom flask). For instance, S2 insect cells are induced with CuSO₄ (e.g. 0.5 mM). After several days (e.g. 4 days), the conditioned culture medium is loaded onto a Protein A Sepharose column and the Fc-Necl-3 or Fc-Necl-4 fusion protein is recovered according to general methods.

Typically, when the Necl-3 and/or Necl-4 antagonists are antibodies to Necl-3/Necl-4 according to the invention, they may be prepared according to known processes.

Methods of preparing polyclonal antibodies from various species have been described for instance in Vaitukaitis et al., 1971, J Clin Endocrinol Metab., 33, 988*.* Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include Necl-3 or Necl-4 or the extracellular fragment or a fragment thereof or a variant as described here above or a fusion protein thereof, or a synthetic peptide encoding a region of Necl-3 or Necl-4. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). Repeated injections may be performed. Blood samples are collected and immunoglobulins or serum are separated.

Methods of producing monoclonal antibodies may be found, for instance, in Kohler et al., 1975, Nature, 256, 495 incorporated therein by reference in its entirety.

The monoclonal antibodies may also be made by recombinant DNA methods, such as those described in US 4,816,567.

Typically, the preparation of the polyclonal antibodies according to the invention is performed by using a fragment of the extracellular domain of Necl-3 or Necl-4 (e.g. the Necl-3₍ₐₐ₁₆₉₋₃₆₇₎ fragment of SEQ ID NO: 15 or Necl-4₍ₐₐ₁₂₆₋₃₂₂₎ of SEQ ID NO: 26) produced in *E. coli* to immunize rabbits. The same fragment is coupled to NHS activated Sepharose 4 Fast Flow and used for affinity purification of the antibodies from the rabbit sera. The antibody-containing fractions are then loaded onto a Protein A Sepharose CL-4B column and the IgGs are eluted.

Typically, the efficacy of the Necl-3/Necl-4 antagonists according to the invention may be assayed in various cell adhesion assays such as described in Biederer et al., 2002, Science. 297:1525-31 or according to the following general process: S2 Drosophila cells are maintained in appropriate medium such as Schneider's Drosophila Medium (Gibco) supplemented with 10% FCS and transfected with an appropriate transfection agent (e.g. Lipofectamine 2000 (Invitrogen), Fugene HD (Roche), or other). After transfection (e.g. about 24 hours) the medium is changed and CuSO₄ is added (e.g. to 0.5 mM) for another period of time (e.g. 24 hours). For aggregation assays, the cells to be tested are mixed after the medium change, induced with CuSO₄ and gently shaken for several hours to allow for plasmid transcription, protein accumulation and aggregate formation. The Necl-3/Necl-4 antagonists and appropriate controls such as unrelated Fc fusion proteins or immunoglobulins are added prior to induction with CuSO₄ and their inhibitory effect on aggregate formation is assessed by microscopy.

In one embodiment, the invention provides an isolated polypeptide having at least 80% (such as at least 85%, at least 90%, at least 95%, at least 98%) identity or homology with a sequence of amino acids selected from the group consisting of:
(a) amino acids 1 to 435 of SEQ ID NO: 2;
(b) amino acids 1 to 365 SEQ ID NO: 4;
(c) amino acids 1 to 388 of SEQ ID NO: 6;
(d) amino acids 1 to 323 of SEQ ID NO: 8;
(e) amino acids 1 to 198 of SEQ ID NO: 15; and
(f) amino acids 1 to 197 of SEQ ID NO: 26.

In a further embodiment, the invention provides an isolated polypeptide according to the invention wherein the isolated polypeptide binds to at least one Necl protein or one of its ligand such as Necl-1, Necl-2, Necl-3 or Nec-4 or fragment thereof In a particular embodiment, the isolated polypeptide according to the invention binds to the extracellular fragment or a fragment thereof of at least one Necl protein, such as Necl-1, Necl-2, Necl-3 or Nec-4.

In a further embodiment, the invention provides an isolated polypeptide according to the invention comprising an amino acid sequence selected from the group consisting of:
SEQ ID NO: 2; SEQ ID NO: 4 and SEQ ID NO: 15. In a further embodiment, the isolated peptide comprises an amino acid sequence of SEQ ID NO: 4.

In another further embodiment, the invention provides an isolated polypeptide according to the invention comprising an amino acid sequence selected from the group consisting of:
SEQ ID NO: 6; SEQ ID NO: 8 and SEQ ID NO: 26. In a further embodiment, the isolated peptide comprises an amino acid sequence of SEQ ID NO: 8.

In another embodiment, the invention provides an isolated nucleic acid consisting of a nucleotide sequence encoding a polypeptide having at least 80% (such as at least 85%, at least 90%, at least 95%, at least 98%) identity or homology with a sequence of amino acids selected from the group consisting of:
(a) amino acids 1 to 435 of SEQ ID NO: 2;
(b) amino acids 1 to 365 SEQ ID NO: 4;
(c) amino acids 1 to 388 of SEQ ID NO: 6;
(d) amino acids 1 to 323 of SEQ ID NO: 8;
(e) amino acids 1 to 198 of SEQ ID NO: 15; and
(f) amino acids 1 to 197 of SEQ ID NO: 26.

In a further embodiment, the invention provides an isolated nucleic acid consisting of a nucleotide sequence encoding a polypeptide having a sequence of amino acids selected from the group consisting of:
(a) amino acids 1 to 435 of SEQ ID NO: 2;
(b) amino acids 1 to 365 of SEQ ID NO: 4;
(c) amino acids 1 to 388 of SEQ ID NO: 6;
(d) amino acids 1 to 323 of SEQ ID NO: 8;
(e) amino acids 1 to 198 of SEQ ID NO: 15; and
(f) amino acids 1 to 197 of SEQ ID NO: 26.

In another embodiment, the invention provides a fusion protein comprising the following sequences:
- an effector region having at least 80% identity or homology (such as at least 85%, at least 90%, at least 95%, at least 98%) with a sequence of amino acids selected from the group consisting of an extracellular domain of said Necl-3 or Necl-4 or a fragment thereof and
- an immunoglobulin constant region (Fc domain or an Fc variants thereof).

In another embodiment, the invention provides a fusion protein comprising the following sequences:
- an effector region having at least 80% identity or homology (such as at least 85%, at least 90%, at least 95%, at least 98%) with a sequence of amino acids selected from the group consisting of SEQ ID NO: 4 and SEQ ID NO: 15; and
- an immunoglobulin constant region (Fc domain or an Fc variants thereof).

In another embodiment, the invention provides a fusion comprising the following sequences:
- an effector region having at least 80% identity or homology (such as at least 85%, at least 90%, at least 95%, at least 98%) with a sequence of amino acids of SEQ ID NO: 8 and SEQ ID NO: 26; and
- an immunoglobulin constant region (Fc domain or an Fc variants thereof).

In another further embodiment, the invention provides a fusion protein according to the invention wherein the effector region is a protein with a sequence of amino acid of SEQ ID NO:4.

In another further embodiment, the invention provides a fusion protein according to the invention wherein the effector region is a protein with a sequence of amino acid of SEQ ID NO:8.

In a further embodiment, the invention provides a fusion protein according to the invention wherein the immunoglobulin constant region is the Fc domain of an IgG.

In another further embodiment, the invention provides a fusion protein according to the invention wherein the Fc domain is the Fc domain of an IgG1 or an IgG4.

In another further embodiment, the invention provides a fusion protein according to the invention wherein the fusion protein is selected from the following group:
The fusion protein formed by the human Necl-3 extracellular domain (aa 1-365) of SEQ ID NO: 4 fused to the human Fc domain of an IgG1 of SEQ ID NO: 35;
The fusion protein formed by the human Necl-3 extracellular domain (aa 169-367) of SEQ ID NO: 15 fused to the human Fc domain of an IgG1 of SEQ ID NO: 35;
The fusion protein formed by the human Necl-3 extracellular domain (aa 1-365) of SEQ ID NO: 4 fused to the human Fc domain of an IgG4 of SEQ ID NO: 36;
The fusion protein formed by the human Necl-3 extracellular domain (aa 1-365) of SEQ ID NO: 4 fused to the mouse Fc domain of an IgG2a of SEQ ID NO: 37;
The fusion protein formed by the human Necl-3 extracellular domain (aa 1-365) of SEQ ID NO: 4 fused to the mouse Fc domain of an IgG3 of SEQ ID NO: 38;
The fusion protein formed by the human Necl-4 extracellular domain (aa 1-323) of SEQ ID NO: 8 fused to the human Fc domain of an IgG1 of SEQ ID NO: 35;
The fusion protein formed by the human Necl-4 extracellular domain (aa 1-323) of SEQ ID NO: 8 fused to the human Fc domain of an IgG4 of SEQ ID NO: 36;
The fusion protein formed by the human Necl-4 extracellular domain (aa 1-323) of SEQ ID NO: 8 fused to the mouse Fc domain of an IgG2a of SEQ ID NO: 37; and
The fusion protein formed by the human Necl-4 extracellular domain (aa 1-323) of SEQ ID NO: 8 fused to the mouse Fc domain of an IgG3 of SEQ ID NO: 38.

In another further embodiment, the invention provides a fusion protein according to the invention wherein the fusion protein is selected from the following group:
The fusion protein formed by the human Necl-3 extracellular domain (aa 1-365) of SEQ ID NO: 4 fused to the human Fc domain of an IgG1 of SEQ ID NO: 35;
The fusion protein formed by the human Necl-3 extracellular domain (aa 1-365) of SEQ ID NO: 4 fused to the human Fc domain of an IgG4 of SEQ ID NO: 36;
The fusion protein formed by the human Necl-4 extracellular domain (aa 1-323) of SEQ ID NO: 8 fused to the human Fc domain of an IgG1 of SEQ ID NO: 35; and
The fusion protein formed by the human Necl-4 extracellular domain (aa 1-323) of SEQ ID NO: 8 fused to the human Fc domain of an IgG4 of SEQ ID NO: 36.

In a particular embodiment, the fusion protein according to the invention of the invention has an inhibitory activity to a Necl protein such as Necl-1 or Necl-2 or Necl-3 or Necl-4 or a ligand thereof.

In another embodiment, the invention provides an isolated DNA sequence that encodes a fusion protein according to the invention.

In a further embodiment, the invention provides an isolated antibody or an immunologically active fragment of a monoclonal antibody that binds to at least one Necl protein selected from Necl-3 and Necl-4.

In another further embodiment, the invention provides an isolated antibody specifically directed against a polypeptide having a sequence of amino acids selected from the group consisting of:
(a) amino acids 1 to 435 of SEQ ID NO: 2;
(b) amino acids 1 to 365 of SEQ ID NO: 4;
(c) amino acids 1 to 388 of SEQ ID NO: 6;
(d) amino acids 1 to 323 of SEQ ID NO: 8;
(e) amino acids 1 to 198 of SEQ ID NO: 15; and
(f) amino acids 1 to 197 of SEQ ID NO: 26.

In another embodiment, the invention provides a use of an antibody according to the invention for the analysis of Necl-3 or Necl-4 expression such as in a western blot, an immunohistochemistry, an ELISA or a FACS assay.

In another embodiment, the invention provides a kit comprising at least one Necl-3 antagonist and/or Necl-4 antagonist according to the invention. More especially, the invention provides a kit comprising at least one Necl-3 antagonist and/or Necl-4 antagonist according to the invention wherein the Necl-3 antagonist and/or Necl-4 antagonist is a fusion protein according to the invention. The kit according to the invention may be used for measuring the activity/or the presence of at least one Necl protein such as Necl-1, Necl-2, Necl-3 or Necl-4 or its ligand. In a particular embodiment, the kit is used for measuring the activity/or the presence of Necl-3 and/or Necl-4.

In another embodiment, the invention provides a recombinant expression vector comprising a nucleic acid molecule according to the invention, wherein the vector optionally comprises an expression control sequence, allowing expression in prokaryotic or eukaryotic host cells of the encoded polypeptide, operably linked to the nucleic acid molecule.

In another embodiment, the invention provides a host cell (e.g. prokaryotic or eukaryotic) transfected or transformed with a recombinant expression vector or a nucleic acid according to the invention.

In another embodiment, the invention provides a process for producing cells capable of expressing a polypeptide according to the invention, comprising genetically engineering cells with a vector or a nucleic acid according to the invention

In another embodiment, the invention provides a process for producing a polypeptide according to the invention, comprising transfecting an expression vector according to the invention into a host cell according to the invention under conditions allowing the expression of said polypeptide according to the invention and recovering the said polypeptide.

In another embodiment, the invention provides a process for producing a fusion protein according to the invention, comprising culturing a host cell transformed with an expression vector according to the invention under conditions that promotes expression of said fusion protein and recovering said fusion protein.

In another embodiment, the invention provides a pharmaceutical composition comprising a Necl-3 antagonist or a Necl-4 antagonist according to the invention, and a physiologically acceptable carrier, diluent or excipient.

In particular, the invention provides a pharmaceutical composition wherein the Necl-3 antagonist or the Necl-4 antagonist is selected from a fusion protein according to the invention and a Necl-3 or a Necl-4 specific binding agent of the invention and a physiologically acceptable carrier, diluent or excipient. More especially, the invention provides a pharmaceutical composition comprising a Necl-3 antagonist or a Necl-4 antagonist according to the invention, wherein the Necl-3 antagonist or Necl-4 antagonist is a fusion protein according to the invention, and a physiologically acceptable carrier, diluent or excipient.

In another embodiment, the invention provides a Necl-3 antagonist or a Necl-4 antagonist for use as a medicament. In particular, the Necl-3 antagonist or the Necl-4 antagonist according to the invention for use as a medicament is selected from a fusion protein according to the invention, an isolated Necl-3 specific binding agent or a Necl-4 specific binding agent according to the invention, and an antibody according to the invention.

In another embodiment, the invention provides a use of a Necl-3 antagonist or a Necl-4 antagonist according to the invention for the manufacture of a medicament for the treatment of CNS disorders comprising neuroinflammatory and neurodegenerative conditions or diseases such as demyelinating disorders or diseases. In particular, the Necl-3 antagonist or the Necl-4 antagonist according to the invention is selected from a fusion protein according to the invention, an isolated Necl-3 specific binding agent or a Necl-4 specific binding agent according to the invention, and an antibody according to the invention.

In a further embodiment, the Necl-3 antagonist or the Necl-4 antagonist according to the invention is a fusion protein according to the invention.

In a further embodiment, the Necl-3 antagonist or the Necl-4 antagonist according to the invention is an antibody or an immunologically active fragment of a monoclonal antibody that binds Necl-3 (Necl-4) and/or blocks the binding of Necl-3 (Necl-4) to other binding partners such as Necl-1, Necl-2, Necl-3 and/or Necl-4

In another embodiment, the invention provides a use of a Necl-3 antagonist or a Necl-4 antagonist according to the invention for the manufacture of a medicament for the treatment of CNS disorders comprising neuroinflammatory and neurodegenerative conditions or diseases such as demyelinating disorders or diseases wherein the Necl-3 antagonist or the Necl-4 antagonist is to be administered in combination with a co-agent that useful in the treatment of CNS disorders, including neuroinflammation and/or neurodegeneration such as a co-agent useful in the treatment of demyelination. In a particular embodiment, the Necl-3 antagonist or a Necl-4 antagonist according to the invention and the co-agent are used simultaneously or sequentially.

In a further embodiment, the invention provides a use of a Necl-3 antagonist or a Necl-4 antagonist according to the invention for the manufacture of a medicament for the treatment of multiple sclerosis.

In a further embodiment, the invention provides a use of a Necl-3 antagonist or a Necl-4 antagonist according to the invention for the manufacture of a medicament for the treatment of CNS disorders comprising neuroinflammatory and neurodegenerative conditions or diseases such as demyelinating disorders or diseases, wherein the Necl-3 antagonist or Necl-4 antagonist is a fusion protein according to the invention.

In another embodiment, the invention provides a method of treating of a disease comprising the administration of a therapeutically effective amount of a Necl-3 antagonist or a Necl-4 antagonist according to the invention in a mammal in need thereof and wherein the disease is a CNS disorders comprising neuroinflammatory and neurodegenerative conditions or diseases such as demyelinating disorders or diseases. In a further embodiment, the mammal is human. In another further embodiment, the human suffers from multiple sclerosis.

The invention relates to methods of promoting remyelination of nerve cells in a mammal comprising administering to the mammal a Necl-3 and/or Necl-4 antagonist according to the invention in a remyelination effective amount. In a particular embodiment, the mammal in the methods of the present invention is a human and the human suffers from a condition that demyelinates cells.

In another embodiment, the invention provides a method of antagonizing the aggregation of Necl-3 and/or of Necl-4 with itself of with at least one protein of the Necl/SynCAM family, comprising exposing cells that express Necl-3 or Necl-4, respectively to a Necl-3 antagonist and/or Necl-4 antagonist according to the invention, such that the Necl-3 antagonist or the Necl-4 antagonist blocks the aggregation of Necl-3 or of Necl-4 with itself or with at least one protein of the Necl/SynCAM family. More particularly, the Necl-3 antagonist or Necl-4 antagonist used in this method is a fusion protein according to the invention.

In another embodiment, the invention provides a method for *in vitro* detection of the level of a Necl-3 and of Necl-4 protein in a biological sample comprising contacting said biological sample with an antibody according to the invention.

In another embodiment, the invention provides a method for screening for a Necl-3 and/or a Necl-4 antagonist, comprising the following steps:
(i) Combining cells that do not aggregate spontaneously but in presence of a Necl-3 and/or a Necl-4 protein with at least one peptide according to the invention, in presence/in absence of a compound to be screened;
(ii) Determining the ability of the compound to inhibit the Necl-3 and/or Necl-4 induced cell aggregation.

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description and accompanying drawings. Such modifications are intended to fall within the scope of the appended claims.

The invention having been described, the following examples are presented by way of illustration, and not limitation.

### Examples

The following abbreviations refer respectively to the definitions below:
**aa** (amino acid); **Da** (Dalton), **Kb** (Kilobase), **µl** (microliter), **mL** (milliliter), **mM** (millimolar), **min** (minute), **mW** (MilliWatt), **nm** (nanometer), **APC** (Antigen Presenting Cell), **BSA** (Bovine Serum Albumin), **CAM** (Cell Adhesion Molecule), **CHO** (Chinese Hamster Ovary), **CNS** (Central nervous system), **CSF** (Cerebrospinal fluid), **DMEM** (Dulbecco's Modified Eagle Medium), **DsRed2** (Discosoma Red), **EDSS** (Expanded Disability Status Scale), **FACS** (Fluorescence Activated Cell Sorte); **FCS** (Fetal calf serum), **GADPH** (Glyseraldehyde-3-phosphate dehydrogenase), **GFP** (Green Fluorescent Protein), **IFN** (interferon), **Ig** (Immunoglobulin), **IPTG** (isopropyl-beta-D-thiogalactopyranoside), i.v. (intra-venous), **MBP** (myelin basic protein), **MHC** (Major Histocompatibility Complex), **MRI** (Magnetic resonance imaging), **MS** (multiple sclerosis), **NHS** (N-hydroxysuccinimide), **PCR** (Polymerase Chain Reaction), **PML** (progressive multifocal leukoencephalopathy), **PPMS** (Primary progressive multiple sclerosis), **PRMS** (Progressive relapsing multiple sclerosis), **RIPA** (Radioimmunoprecipitation assay), **RRMS** (Relapsing-remitting multiple sclerosis), **SDS** (Sodium Dodecyl Sulfate), **SNRS** (Scripps Neurologic Rating Scale), **SPMS** (Secondary progressive multiple sclerosis), **SynCAM** (Synaptic CAM), **s.c.** (subcutaneous).

### Example 1: Necl-3 expression vectors

Amplifications were performed by PCR reaction with Pfu Turbo polymerase (Stratagene) according to the supplier's instructions (1X cloned *Pfu* buffer, 200 µM each dNTP, 0.1-0.5 µM each primer, 2.5 units cloned enzyme, 1-100 ng plasmid template DNA, or 100-250 ng genomic template DNA).

Mammalian expression vectors pNecl-3-GFP and pNecl3-DsRed2 were constructed as follows:
- the cDNA DKFZp761G128 (accession AL834270) of SEQ ID NO: 9 was used as a template for PCR amplification with the primers 5'-GGAATTCATGATTTGGAAACGC-3' of SEQ ID NO: 10 and 5'-CCCCAATTGCAATGAAATACTCTTT-3 of SEQ ID NO: 11, respectively.
- the resulting amplicon was digested with EcoR1 and Mfe1 and ligated into the EcoR1 site of pEGFP-N1 and pDsRed2-N1 (BD Biosciences Clonetech).

Another mammalian expression vector pNecl-3-myc was constructed as follows:
- the cDNA DKFZp761G128 of SEQ ID NO: 9 was used as a template for PCR amplification with the primers 5'-GGAATTCATGATTTGGAAACGC-3' of SEQ ID NO: 10 and 5'-CCCCAATTGCAATGAAATACTCTTT-3 of SEQ ID NO: 11.
- the resulting amplicon was digested with EcoR1 and Mfe1 and ligated into the EcoR1 site of pcDNA3.1/myc-His (Invitrogen).
- The insect cell expression vector pMT-Necl-3-GFP and pMT-Necl-3-DsRed2 were constructed as follows: the pNecl-3-GFP and pNecl-3-DsRed2 vectors were digested with EcoR1 and Not1 and the resulting inserts were ligated into the plasmid pMT (Invitrogen), digested with the same enzymes.

The resulting expression vectors were transfected into S2 Drosophila cells that were maintained in Schneider's Drosophila Medium (Gibco) supplemented with 10% FCS. The transfection was performed with Lipofactamine 2000 (Invitrogen) in 6-well culture plates: 2 µg of DNA were diluted in 50 µl of medium without serum. Lipofectamine 2000 was mixed gently before use, then diluted in 50 µl of medium, and incubated for 5 minutes at room temperature. After 5 minutes of incubation the diluted DNA was diluted in Lipofectamine 2000 (total volume =100 µl), mixed and incubated for 20 minutes at room temperature. The 100 µl of complexes were added to each well containing cells and medium. 24 hours after transfection the medium was changed and 0.5 mM CuSO₄ was added for another 24 hours.

For aggregation assays, cells to be tested were mixed after the medium change, then induced with CuSO₄ and gently shaken several hours to allow for plasmid transcription, protein accumulation and aggregate formation. Hela cells were maintained in DMEM 10% FCS and transfected with Fugene (Roche) Fugene was mixed gently before use, then diluted in 100 µl of medium without serum, and incubated for 5 minutes at room temperature. After 5 minutes of incubation 2 µg of DNA were added to the diluted Fugene, mixed and incubated for 20 minutes at room temperature. The 100 µl of complexes were added to each well containing cells and medium.

### Example 2: Necl-1 and Necl-2 expression vectors

The amino acid sequence encoding human Necl-1 and -2, are presented in SEQ ID NO: 39 and SEQ ID NO: 40, respectively.

The expression vectors of the Necl-1 and Necl-2 protein fused with GFP or with DsRed2 (respectively pMT-Necl-1-GFP/pMT-Necl-1-DsRed2 and pNecl-2-GFP which were used in the aggregation assay described in Example 6 were constructed as follows:
The cDNA IMAGE: 5199627 (accession BC033819) of SEQ ID NO: 29 was used as a template for PCR amplification with the primers 5'-CCCCAATTGATGGGGGCCCCAGCCGC -3' of SEQ ID NO: 30 and 5'-CCCCAGATCTAAGATGAAATATTCCTTCTTGTCGTCCCC -3 of SEQ ID NO: 31. The resulting amplicon was digested with Bg12 and Mfe1 and ligated into the EcoR1 and BamH1 sites of pMT-Necl-3-GFP resulting in the pMT-Necl-1-GFP. The same digested amplicon was ligated into the EcoR1 and BamH1 sites of pMT-Necl-3-DsRed2 resulting in the pMT-Necl-1-DsRed2.
The cDNA IMAGE: 4701395 (accession BC035930) of SEQ ID NO: 32 was used as a template for PCR amplification with the primers 5'- GGAATTCATGGCGAGTGTAGTG - 3' of SEQ ID NO: 33 and 5'- CCCCAATTGCGATGAAGTACTCTTT -3 of SEQ ID NO: 34. The resulting amplicon was digested with EcoR1 and Mfe1 and ligated into the EcoR1 site of pEGFP-N1 (BD Biosciences Clonetech) resulting in pNecl-2-GFP. The insect vector pMT-Necl-2-GFP was constructed as follows: the pNecl-2-GFP vector was digested with EcoR1 and Not1 and the resulting inserts were ligated into the plasmid pMT (Invitrogen), digested with the same enzymes.

### Example 3: Expression of an extracellular domain of Necl-3 (SEQ ID NO: 14)

A bacterial expression vector pQE-Necl-3₍ₐₐ₁₆₉₋₃₆₇₎ was constructed as follows:
- the plasmid pNecl-3-myc obtained under Example 1 was used as a template for PCR amplification with the primers 5'-AGGTCTATATAAGC-3' of SEQ ID NO: 12 and 5'-GGGGTACCAGGGCCATTCTGGCC-3 of SEQ ID NO: 13
- the resulting amplicon was digested with Dra1 and Kpn1 and ligated into the blunted BamH1 and Kpn1 sites of the plasmid pQE-30 (Qiagen).

The *E. coli* strain M15 was transformed with the bacterial expression vector pQE-Necl-3₍ₐₐ₁₆₉₋₃₆₇) and used to express the corresponding Necl-3 fragment. Bacteria were grown exponentially, IPTG was added to 1 mM, and the cells were allowed to grow for another 4 h at 37°C. Cells were then harvested and lysed. The cleared supernatants were loaded under denaturing conditions onto Ni²⁺-nitrilotriacetic acid agarose columns (Qiagen, 1 mL resin per 200 mL of bacteria culture), and eluted under standard conditions. The eluted proteins were then extensively dialyzed against PBS and used for immunization.

The obtained Necl-3₍ₐₐ₁₆₉₋₃₆₇₎ fragment has an amino acid sequence of SEQ ID NO: 15.

### Example 4: Raising of antibodies against an extracellular domain of Necl-3 (SEQ ID NO: 15)

The Necl-3₍ₐₐ₁₆₉₋₃₆₇₎ fragment produced in *E*. *coli* as described under Example 3 and having the sequence SEQ ID NO: 15 was used to immunize two rabbits according to Harlow et al., 1988, "Antibodies: A Laboratory Manual, " Cold Spring Harbor Laboratory, Cold Spring Harbor, NY, 349*.*

This fragment was coupled to NHS activated Sepharose 4 Fast Flow (Amersham Biosciences) and used for affinity purification of the anti Necl-3 antibodies from the rabbit sera: 10 mg of protein was used for 2 mL of resin. Coupling was performed at 4 °C overnight. After the coupling, non-reacted groups on the medium were blocked with 0.5 M ethanolamine, 0.5 M NaCl, pH 8.3 for a one hour. The medium was washed after coupling with 50 mL of two buffers (0.1 M Tris-HCl buffer pH 8-9 and 0.1 M acetate buffer, 0.5 M NaCl). The antibody-containing fractions were then loaded onto a Protein A Sepharose CL-4B column (Amersham Biosciences) and the IgGs were eluted with 0.1 M glycine buffer pH 3.0).

The antibody specificity was tested using *Drosophila* S2 cells transfected with either GFP alone or with Necl-1, Necl-2 (obtained as described in Example 2), Necl-3 (as described in Example 1) and Necl-4 (as described in Example 7) fused to GFP at their carboxy-termini where crude S2 lysates were separated by SDS gel electrophoresis and probed with either the anti Necl-3 (obtained as described in Example 4) or an anti GFP antibody (anti-green fluorescent protein, Roche Applied Science) serving as a loading control. The anti Necl-3 antibody reveals a single species of the expected size exclusively in cells transfected with Necl-3-GFP which proves that highly specific anti Necl-3 antibodies have been prepared which do not cross-react with other Necl/SynCAM family members, i.e. Necl-1, Necl-2, or Necl-4.

### Example 5: Localization of Necl-3 adhesion molecules

Necl-3 mRNA expression in eighteen various rat tissues was assessed by real time PCR using a primer pair spanning two exons. The primers used for amplifying Necl-3 were SEQ ID NO: 16 (5'-TGACCATGCTCTCATAGG-3') and SEQ ID NO: 17 (5'-TGCCAGATATCGACCAAG-3'), those for GAPDH were SEQ ID NO: 18 (5'-TGATTCTACCCACGGC-3') and SEQ ID NO: 19 (5'-TGATGGGTTTCCCATTGATGA-3').

The results confirmed northern blot data showing a Necl-3 mRNA greater than 5 Kb in various structures of the central nervous system (midbrain, cerebellum, and hippocampus), whereas it was either undetectable or poorly expressed in all other organs tested except for testis. Real time PCR analysis were performed with SYBR green (Molecular Probes) on an iCycler iQ Bio-Rad station, with quantitation, melt curve, and PCR efficiency analysis derived by the station's software as described in Pellissier et al., 2006, Anal. Biochem, 350:310-2 incorporated by reference in its entirety.

RNA purification and cDNA synthesis were prepared as described in *(*Ossipow et al., 2004, Mol Cell Neurosci. 2 7:70-83 *).*
Necl-3 protein accumulation was examined by using Necl-3 antibodies obtained as described in Example 4. Western blot analysis with extracts from a series of different organs was carried out where rat tissues were heated and sonicated in Laemmli sample buffer. Proteins were separated on a 12% SDS-polyacrylamide gels, and transferred to nitrocellulose membranes. These were probed with the appropriate antibodies and revealed by chemiluminescence (ECL, Amersham) (equal amounts of the ECL substrates were mixed and incubated with the blot for 5 min). Necl-3 protein was detected in brain structures, but also in kidney, heart and liver. Necl-3 migrates with an apparent mass of approximately 52 kDa (calculated molecular weight of 48 kDa), due to N-linked glycosylation.

The brain distribution of Necl-3 was investigated by immunohistochemistry and confocal microscopy. 2 month-old rats were anaesthetized, their brains were rapidly excised and immersed for 5 min in isopentane pre-cooled to -20°C. Brains were then embedded in OCT medium (Miles Inc., Elkhart, IN) and cryo-sectioned at 12-µm.

Brain sections were exposed for 10 min to -20°C absolute ethanol, rinsed in PBS and incubated for 30 min in PBS containing 2% BSA. Sections were then incubated for 2 h at 37°C in PBS, 0.5% BSA, containing the affinity-purified anti-Necl-3 antibody (diluted 1:100) obtained as described under Example 4 and the appropriate mouse monoclonal antibody. After washes in PBS, Alexa Fluor highly cross-adsorbed secondary antibodies (Molecular Probes) were used to detect the mouse and rabbit primary antibodies (with Alexa 488 and Alexa 594, respectively). The brain sections were then rinsed again in PBS and stained with Hoechst. Confocal laser scanning was performed on a Leica SP2 microscope (Leica, Germany) using 20 mW blue diode (405 nm), 30 mW ArKr (488 nm), 1 mW HeNe (594 nm) lasers and a 63X plan Apo oil immersion objective. Section planes were collected in steps throughout the entire cell thickness. Images of each field were merged using the Leica Confocal software. Z-section analysis was done with the Imaris software (Bitplane AG, Switzerland) using the "Section" function with the stacks series of confocal acquisitions.

Intense labeling was observed in structures resembling axon bundles in several brain areas including cerebellar white matter and ventral reticular nucleus. The axonal localization of Necl-3 was confirmed by a near-perfect overlap with the axonal neurofilament marker.

Monoclonal antibodies directed against the MBP (Calbiochem) were used in combination with anti Necl-3 antibodies obtained as described under Example 4 to confirm that Necl-3 is found in the axoplasm of myelinated axons and that Necl-3 immunoreactivity is stronger in the myelinated areas.

### Example 6: Aggregation of Necl-3 molecules- Fusion protein preparation

S2 cells do not aggregate spontaneously and the ability of Necl-3 to induce their aggregation was assayed. S2 cells were transfected separately with plasmids expressing GFP (the insect cell expression vector pMT-GFP was constructed as follows: pEGFP-N1 (BD Biosciences Clonetech) was digested with EcoR1 and Not1, and the resulting insert was ligated into the plasmid pMT (Invitrogen), digested with the same enzymes) or Necl-3-GFP obtained under Example 1 under the control of the drosophila inducible metallothionein promoter. After transfection, the cells were washed free from plasmid, and mixed together. They were then induced with CuSO₄, gently shaken to favor aggregation and visualized without further fixation. Unlike cells transfected with GFP, those transfected with Necl-3-GFP formed large aggregates. Non-transfected cells serving as internal control did not aggregate.

S2 cells were separately transfected with plasmids coding for Necl-3-GFP (Example 1), Necl-1-DsRed2 (Example 2), and Necl-4-DsRed2 (Example 7). After transfection, they were mixed together in different combinations and induced to express their transfected Necl. After gentle shaking, aggregates formed between Necl-3-GFP and Necl-1-DsRed2 or Necl-4-DsRed2 were observed by fluorescence microscopy. A similar S2 cells aggregation assay using a myc-tagged version of Necl-3 (pNecl-3-myc) along with Necl-2-GFP. Using a rhodamine-conjugated anti myc secondary antibody, aggregates of red and green cells were again observed.

Thus, Necl-3 unambiguously interacts with itself, Necl-1, Necl-2, and Necl-4.

An Fc-Necl-3 fusion protein between the Fc domain of a human IgG1 of SEQ ID NO: 35 and the extra-cellular domain of Necl-3 (aa 1-365) of SEQ ID NO: 2 was prepared as follows: First, a mammalian expression vector pFc-Necl-3 was constructed by using the plasmid pNecl-3-myc (Example 1) as a template for PCR amplification with the primers 5'-AGGTCTATATAAGC-3' of SEQ ID NO: 12 and 5'-GGGGTACCAGGGCCATTCTGGCC-3 of SEQ ID NO: 13. The resulting amplicon was digested with Nhe1 and Kpn1 and ligated into the plasmid pIgplus (R&D Systems, Abingdon, United Kingdom) digested with the same enzymes, resulting in pFc-Necl-3. An insect cell expression vector pMT-Fc-Necl-3 was then constructed wherein the pFc-Necl-3 vector obtained above was digested with Nhe1 and Apa1 and the resulting insert was ligated into the plasmid pMT (Invitrogen) digested with Spe1 and Apa1 resulting in pMT-Fc-Necl-3.

The obtained insect cell expression vector pMT-Fc-Necl-3 was then co-transfected in S2 cells along with the plasmid pCoBlast that carries a blasticidin S resistance gene (Invitrogen). Stable clones were selected with blasticidin S at 25 µg/mL. Of these, high expressers of the Fc-Necl-3 fusion were selected. One such clone was expanded and grown in a 150 mL flat bottom flask and induced with 0.5 mM CuSO₄. After 4 days, the conditioned culture medium was loaded onto a Protein A Sepharose CL-4B column (Amersham Biosciences). The Fc-Necl-3 fusion protein which was bond to the resin directly from the culture medium was recovered by washing the column (10 volumes of Tris-HCl: 50 mM, pH 7.4) and eluting (0.1 M glycine buffer pH 3.0), and was then characterised by western blotting.

This fusion protein or a control full-length human IgGs (Sigma) were adsorbed on protein A resin. 100µl of lysates of mock-transfected and pMT-Necl-2-GFP transfected S2 cells (Example 2) were pre-cleared for 30 min at 4°C with 25 µl of Protein A Sepharose CL-4B resin and used as the inputs for the following pull-down experiments:

After pre-clearing, the lysates were rocked 45 min at 4°C with the Fc-Necl-3 fusion protein immobilized on Protein A Sepharose CL-4B resin. After incubation, the proteins retained on the beads were washed 3 times with 500 µl RIPA buffer, and boiled in 1X Laemmli sample buffer for western blot analysis. After incubation and washings, the proteins retained on the beads were analyzed by western blot. Control IgG beads were unable to bring down Necl-2-GFP, whereas the Fc-Necl-3 beads efficiently did so. This shows that Necl-3 is able to make heterophilic interactions with Necl-2, and the fact that human IgGs beads do not interact with Necl-2 confirms that even though the Necl proteins are capable of multiple heterophilic interactions, they retain specificity.

### Example 7: Necl-4 expression vector

Necl-4 amplification was performed by PCR reaction with Pfu Turbo polymerase (Stratagene): 1X cloned *Pfu* buffer, 200 µM each dNTP, 0.1-0.5 µM each primer, 2.5 units cloned enzyme, 1-100 ng plasmid template DNA, or 100-250 ng genomic template DNA). Insect expression vectors pMT-Necl-4-GFP and pMT-Necl-4-DsRed2 were constructed as follows:
- the modified cDNA clone IMAGE 4375136 (accession BC068457) mutated in position 563 wherein A is mutated in G as shown on SEQ ID NO: 20 was used as a template for PCR amplification with the primers 5'-CCCCAATTGATGGGCCGGGCCCGG-3' of SEQ ID NO: 21 and 5'-CCCCAGATCTAAGATGAAGAATTCCTCTTTCCTCTTGT-3 of SEQ ID NO: 22.
- the resulting amplicon was digested with Mfe1 and Bg12 and ligated into either a pMT-GFP or a pMT-DsRed2 digested by EcoR1 and BamH1.

A mammalian expression vector pNecl-4-GFP was constructed as follows:
- pMT-Necl-4-GFP obtained above was digested with Xba1 and the fragment Necl-4-GFP was ligated into the pcDNA3.1/myc-His (Invitrogen) digested with the same enzyme.

A bacterial expression vector pQE-Necl-4₍ₐₐ₁₂₆₋₃₂₂₎ was constructed as follows:
- the plasmid pNecl-4-GFP above was used as a template for PCR amplification with the primers 5'-CCCGCATGCAATCCTGTGGTGGAGGTC-3' of SEQ ID NO: 23 and 5'-CCCCTGCAGGGGAACCGACGTCTGA-3 of SEQ ID NO: 24.
- the resulting amplicon was digested with Sph1 and Pst1 and ligated into the pQE-30 (Qiagen), digested with the same enzymes.

The insect vectors were transfected into S2 Drosophila cells under the same conditions as described for the transfection conditions described in Example 1.

### Example 8: Expression of Necl-4 an extracellular domain (SEQ ID NO: 26)

A M15 E. coli strain was transformed with the bacterial expression vector pQE-Necl-4₍ₐₐ₁₂₆₋₃₂₂₎ obtained above and used to express the corresponding Necl-4 fragment. The bacteria were grown exponentially, 1 mM IPTG was added, and the cells were allowed to grow for another 4 h at 37°C. Cells were then harvested and lysed. The cleared supernatants were loaded under denaturing conditions onto Ni²⁺-nitrilotriacetic acid agarose columns (Qiagen), i.e. 1 mL resin per 200 mL of bacteria culture, and eluted under standard conditions. The eluted proteins were then extensively dialyzed against PBS and used for immunization. The obtained Necl-4₍ₐₐ₁₂₆₋₃₂₂₎ fragment has an amino sequence of SEQ ID NO: 26.

### Example 9: Raising of antibodies against an extracellular domain of Necl-4 (SEQ ID NO:26)

The Necl-4₍ₐₐ₁₂₆₋₃₂₂₎ fragment produced in *E. coli* as described under Example 7 and having the amino acid sequence of SEQ ID NO: 26 was used by to immunize two rabbits on the same manner as described under Example 4.

### Example 10: Localization of Necl-4 adhesion molecules

Necl-4 expression was examined in various tissues by real-time PCR in the way as described under Example 5 and wherein the primers used for amplifying Necl-4 were 5'-GAGGGCGCTCTACGTACTTG-3' of SEQ ID NO: 27 and 5'-AACCGACGTCTGAGCCTCTA-3' of SEQ ID NO: 28, those for GAPDH were of SEQ ID NO: 18 and SEQ ID NO: 19.

The Necl-4 expression was shown to be the highest in brain and spinal cord.

### Example 11: Aggregation of Necl-4 molecules

The ability of Necl-4 to form homophilic interaction was demonstrated as described under Example 6 wherein the S2 cells were transfected with the plasmid expressing Necl-4-GFP obtained under Example 7.

The ability of Necl-4 to form heterophilic interaction was demonstrated as described under Example 6 wherein the S2 cells were separately transfected with the plasmid expressing Necl-4-GFP obtained under Example 7, the plasmid coding for Necl-3-GFP obtained under Example 1, the plasmids coding for Necl-4-DsRed2 obtained under Example 7, Necl-1-GFP and Necl-2-GFP obtained under Example 2.

Necl-4 showed to unambiguously interact with itself, Necl-1, Necl-2, and Necl-3.

### Example 12: Fusion proteins

The following fusion proteins for modulating the Necl-3 and/or Necl-4 induced cell aggregation are prepared as described in Example 6.

The fusion protein formed by the human Necl-3 extracellular domain (aa 1-365) of SEQ ID NO: 4 fused to the human Fc domain of an IgG1 of SEQ ID NO: 35;

The fusion protein formed by the human Necl-3 extracellular domain (aa 1-365) of SEQ ID NO: 4 fused to the human Fc domain of an IgG4 of SEQ ID NO: 36;

The fusion protein formed by the human Necl-3 extracellular domain (aa 1-365) of SEQ ID NO: 4 fused to the mouse Fc domain of an IgG2a of SEQ ID NO: 37;

The fusion protein formed by the human Necl-3 extracellular domain (aa 1-365) of SEQ ID NO: 4 fused to the mouse Fc domain of an IgG3 of SEQ ID NO: 38;

The fusion protein formed by the human Necl-4 extracellular domain (aa 1-323) of SEQ ID NO: 8 fused to the human Fc domain of an IgG1 of SEQ ID NO: 35;

The fusion protein formed by the human Necl-4 extracellular domain (aa 1-323) of SEQ ID NO: 8 fused to the human Fc domain of an IgG4 of SEQ ID NO: 36;

The fusion protein formed by the human Necl-4 extracellular domain (aa 1-323) of SEQ ID NO: 8 fused to the mouse Fc domain of an IgG2a of SEQ ID NO: 37; and

The fusion protein formed by the human Necl-4 extracellular domain (aa 1-323) of SEQ ID NO: 8 fused to the mouse Fc domain of an IgG3 of SEQ ID NO: 38.

### Example 13: Models for demylinating diseases

The efficiency of the Necl-3 antagonists or Necl-4 antagonists and in particular of fusion proteins according to the invention in the treatment of CNS disorders may be assayed in different models such as Experimental Autoimmune Encephalomyelitis (EAE) model such as described in Loo et al., 2006, Nature Immunology 7(9): 954-961*.* In particular, Necl-3 antagonists or Necl-4 antagonists according to the invention may be subjected to i.v. injection with various doses and dosing regimens in mice primed to develop (EAE). Specifically, a MOG peptide (SEQ ID NO: 41) (Sigma-Aldrich Co.) is injected into mice of 10-15 weeks of age by subcutaneous injection of 200 µg peptide in 200 µl sterile PBS emulsified with an equal volume of complete Freund's adjuvant (Sigma) containing 5 mg/mL of *Mycobacterium tuberculosis* H37Ra (BD). The effect of Necl-3 antagonists or Necl-4 antagonists according to the invention on the disease course are assigned with scores on a scale of 0-6, with 0.5 points for immediate clinical findings, as follows: 0, normal; 1, weakness of the tail; 2, complete loss of tail tonicity; 3, partial hind limb paralysis; 4, complete hind limb paralysis; 5, forelimb paralysis or moribund; and 6, death.

## Claims

1. An isolated polypeptide having at least 80% identity or homology with a sequence of amino acids selected from the group consisting of:
(a) amino acids 1 to 435 of SEQ ID NO: 2;
(b) amino acids 1 to 365 SEQ ID NO: 4;
(c) amino acids 1 to 388 of SEQ ID NO: 6;
(d) amino acids 1 to 323 of SEQ ID NO: 8;
(e) amino acids 1 to 198 of SEQ ID NO: 15; and
(f) amino acids 1 to 198 of SEQ ID NO: 26.

2. An isolated polypeptide according to claim 1 comprising an amino acid sequence selected from the group consisting of: SEQ ID NO: 2; SEQ ID NO: 4; SEQ ID NO: 6; SEQ ID NO: 8; SEQ ID NO: 15 and SEQ ID NO: 26.

3. An isolated nucleic acid consisting of a nucleotide sequence encoding a polypeptide having at least 80% identity or homology with a sequence of amino acids selected from the group consisting of:
(a) amino acids 1 to 435 of SEQ ID NO: 2;
(b) amino acids 1 to 365 SEQ ID NO: 4;
(c) amino acids 1 to 388 of SEQ ID NO: 6;
(d) amino acids 1 to 323 of SEQ ID NO: 8;
(e) amino acids 1 to 198 of SEQ ID NO: 15; and
(f) amino acids 1 to 198 of SEQ ID NO: 26.

4. A fusion protein comprising the following sequences:
- an effector region having a sequence of amino acids according to claims 1 or 2;
- an immunoglobulin constant region.

5. A fusion protein according to claim 4 wherein the effector region is a protein with an amino acid sequence of SEQ ID NO: 4.

6. A fusion protein according to claim 4 wherein the effector region is a protein with an amino acid sequence of SEQ ID NO: 8.

7. A fusion protein according to any of claims 4 to 6 wherein the immunoglobulin constant region is the Fc domain of an IgG.

8. A fusion protein according to any of claims 4 to 7 selected from the following group:
A fusion protein formed by SEQ ID NO: 4 fused to the human Fc domain of an IgG1 of SEQ ID NO: 35;
A fusion protein formed by SEQ ID NO: 4 fused to the human Fc domain of an IgG4 of SEQ ID NO: 36;
A fusion protein formed by SEQ ID NO: 8 fused to the human Fc domain of an IgG1 of SEQ ID NO: 35; and
A fusion protein formed by SEQ ID NO: 8 fused to the human Fc domain of an IgG4 of SEQ ID NO: 36.

9. A fusion protein according to any one of claims 4 to 8 for use as a medicament.

10. A pharmaceutical composition comprising fusion protein according to any one of claims 4 to 8 and a physiologically acceptable carrier, diluent or excipient.

11. Use of a Necl-3 antagonist or a Necl-4 antagonist for the manufacture of a medicament for the treatment of CNS disorders comprising neuroinflammatory and neurodegenerative conditions or demyelinating disorders or diseases.

12. A use according to claim 11 wherein the Necl-3 antagonist or the Necl-4 antagonist is a fusion protein according to any one of claims 4 to 8.

13. A use according to claims 11 or 12 wherein the CNS disorder is multiple sclerosis.

14. A use according to any one of claims 11 to 13 wherein the Necl-3 antagonist or the Necl-4 antagonist is to be administered in combination with a co-agent useful in the treatment of CNS disorders.

15. An isolated DNA sequence that encodes a fusion protein according to any one of claims 4 to 8.

16. A recombinant expression vector comprising a nucleic acid molecule according to claim 3, wherein the vector optionally comprises an expression control sequence, allowing expression in prokaryotic or eukaryotic host cells of the encoded polypeptide, operably linked to the nucleic acid molecule.

17. A host cell transfected or transformed with a recombinant expression vector according to claim 16 or a nucleic acid according to claim 3.

18. A process for producing cells capable of expressing a polypeptide according to claims 1 or 2 or a fusion protein according to any one of claims 4 to 8 comprising genetically engineering cells with a vector according to claim 16 or a nucleic acid according to claim 3.

19. A kit comprising at least one fusion protein according to any one of claims 4 to 8.

20. A process for producing a fusion protein according to any one of claims 4 to 8 comprising culturing a host cell transformed with an expression vector according to claim 16 under conditions that promotes expression of said fusion protein and recovering said fusion protein.

21. A method of antagonizing the aggregation of Necl-3 and/or of Necl-4 with itself of with at least one protein of the Necl family, comprising exposing cells that express Necl-3 or Necl-4, to a fusion protein according to any one of claims 4 to 8, such that the aggregation of said Necl-3 or Necl-4 with itself or with at least one protein of the Necl family is antagonized.

22. A method for screening for a Necl-3 and/or a Necl-4 antagonist comprising the following steps:
(i) Combining cells that do not aggregate spontaneously in presence of a Necl-3 and/or a Necl-4 protein with at least one peptide according to claim 1 or 2, in presence/absence of a compound to be screened;
(ii) Determining the ability of the compound to inhibit the Necl-3 and/or Necl-4 induced cell aggregation.
